# FASCICULE DE BREVET EUROPEEN

(11) **EP 3 943 493 B1**
(45) Date de publication et mention de la délivrance du brevet: **19.04.2023**
(21) Numéro de dépôt: 21190088.1
(22) Date de dépôt: 17.01.2020
(51) Int. Cl.: C07D 471/08, A61K 49/08, A61K 49/10, C07D 487/04

(54) **COMPLEXE DE GADOLINIUM ET D'UN LIGAND CHELATEUR DERIVE DE PCTA DIASTEREOISOMERIQUEMENT ENRICHI ET PROCEDE DE PREPARATION ET DE PURIFICATION**
KOMPLEX AUS GADOLINIUM UND EINEM CHELATBILDENDEN LIGANDEN, DAS VON MIT DIASTEREOMEREN ANGEREICHERTEM PCTA ABGELEITET WIRD, UND ENTSPRECHENDES HERSTELLUNGS- UND REINIGUNGSVERFAHREN
COMPLEX OF GADOLINIUM AND A CHELATING LIGAND DERIVED FROM DIASTEREOISOMERICALLY ENRICHED PCTA AND METHOD FOR PREPARING AND PURIFYING SAME

(30) Priorité: 17.01.2019 FR 1900432
(43) Date de publication de la demande: 26.01.2022
(62) Demande divisionnaire de: 20701709.6
(73) Titulaire: Guerbet, 93420 Villepinte (FR)
(72) Inventeur: LE GRENEUR, Soizic, 91440 Bures sur Yvette (FR); CHÉNEDÉ, Alain, 17140 Lagord (FR); CERF, Martine, 17870 Breuil-Magné (FR); PETTA, Myriam, 95160 Montmorency (FR); MARAIS, Emmanuelle, 78150 Le Chesnay (FR); FRANÇOIS, Bruno, 17170 Saint Jean de Liversay (FR); ROBIC, Caroline, 94130 Nogent sur Marne (FR); LOUGUET, Stéphanie, 94270 Le Kremlin Bicetre (FR)
(74) Mandataire: Regimbeau

(56) Documents cités:
- EP-A1- 1 931 673
- WO-A1-2014/174120
- WO-A1-2020/030618

## Description

La présente invention a trait à une composition comprenant un complexe de gadolinium et d'un ligand chélateur dérivé de PCTA diastéréoisomériquement enrichi en les stéréoisomères dudit complexe qui présentent des propriétés physico-chimiques tout particulièrement intéressantes pour des applications en tant qu'agent de contraste dans le domaine de l'imagerie médicale, notamment pour l'imagerie par Résonance Magnétique.

On connaît de nombreux agents de contraste à base de chélates de lanthanides (métal paramagnétique), en particulier de gadolinium (Gd), décrits par exemple dans le brevet US 4,647,447. Ces produits sont souvent rassemblés sous le terme GBCA (Gadolinium-based Contrast Agent, produits de contraste à base de gadolinium). Plusieurs produits sont commercialisés, parmi lesquels figurent des chélates macrocycliques, tels que le gadotérate de méglumine à base de DOTA (acide 1,4,7,10-tétraazacyclododécane-N,N',N",N"'-tétraacétique), le gadobutrol à base de DO3A-butrol, le gadotéridol à base de HPDO3A, ainsi que des chélates linéaires, notamment à base de DTPA (acide diéthylènetriaminepentaacétique) ou de DTPA-BMA (ligand du gadodiamide).

D'autres produits, dont certains sont en cours de développement, représentent une nouvelle génération de GBCA. Il s'agit essentiellement de complexes de chélates macrocycliques, tels que l'acide bicyclopolyazamacrocyclocarboxylique (EP 0 438 206) ou de dérivés de PCTA (c'est-à-dire comprenant a *minima* la structure chimique de l'acide 3,6,9,15-tétraazabicyclo[9,3,1]pentadéca-1(15),11,13-triène-3,6,9-triacétique), comme décrits dans le document EP 1 931 673.

Les complexes de ligands chélateurs dérivés de PCTA décrits dans le document EP 1 931 673 ont notamment comme avantage d'être relativement faciles à synthétiser chimiquement et, de surcroît, de présenter une relaxivité supérieure aux autres GBCA (relaxivité r₁ pouvant aller jusqu'à 11-12 mM⁻¹.s⁻¹ dans l'eau) actuellement sur le marché, cette relaxivité correspondant à l'efficacité de ces produits et donc à leur pouvoir contrastant.

Dans l'organisme, les chélates (ou complexes) de lanthanide - et notamment de gadolinium - sont en situation d'équilibre chimique (caractérisé par sa constante thermodynamique Kₜₕₑᵣₘ), ce qui peut conduire à une libération non souhaitée dudit lanthanide (voir équation 1 ci-après) :

Ch + Ln ⇄ Ch-Ln (équation 1)

### Equilibre chimique de complexation entre le chélate ou ligand (Ch) et le lanthanide (Lₙ) pour conduire au complexe Ch-Lₙ.

Depuis 2006, une pathologie appelée NSF (Nephrogenic Systemic Fibrosis, fibrose néphrogénique systémique ou dermopathie fibrogénique), a été reliée, au moins en partie, à la libération de gadolinium libre dans l'organisme. Cette maladie a conduit à une alerte des autorités de santé vis-à-vis d'agents de contraste gadolinés commercialisés pour certaines catégories de patients.

Des stratégies ont donc été mises en place pour résoudre de manière complètement sécurisée le problème complexe de la tolérance chez le patient, et limiter, voire éliminer, le risque de libération de lanthanide non souhaitée après administration. Ce problème est d'autant plus délicat à résoudre que l'administration d'agents de contraste est souvent répétée, que ce soit lors d'examens de diagnostic, ou pour l'ajustement des doses et le suivi de l'efficacité d'un traitement thérapeutique.

En outre, depuis 2014 est évoqué un possible dépôt cérébral de gadolinium après administrations répétées de produits gadolinés, plus particulièrement de chélates de gadolinium linéaires, un tel dépôt n'ayant pas, ou peu, été rapporté avec les chélates macrocycliques de gadolinium, comme Dotarem^{®}. En conséquence, différents pays ont décidé soit de retirer la plupart des chélates linaires du marché, soit de limiter drastiquement leurs indications d'utilisation, compte tenu de leur stabilité estimée insuffisante.

Une première stratégie de limitation du risque de libération de lanthanide dans l'organisme consiste ainsi à opter pour des complexes qui se distinguent par des stabilités thermodynamique et/ou cinétique les plus élevées possible. En effet, plus le complexe est stable, plus la quantité de lanthanide libérée au cours du temps sera limitée.

D'autres axes d'amélioration de la tolérance des chélates de lanthanide (notamment de gadolinium) sont décrits dans l'art antérieur. Le document US 5,876,695, datant de plus de trente ans, rapporte par exemple des formulations comprenant, outre le chélate de lanthanide, un agent complexant additionnel, destiné à prévenir une libération *in-vivo* non souhaitée du lanthanide, en venant complexer le lanthanide (ion métallique Gd³⁺) relargué. L'agent chélatant additionnel peut être introduit dans la formulation soit sous sa forme libre, soit sous la forme d'un complexe faible, typiquement de calcium, sodium, zinc ou magnésium. S'il peut, éventuellement, être distinct du ligand constitutif du complexe actif, il importe néanmoins que le complexe qu'il forme avec le lanthanide libéré soit moins stable que le complexe actif, de façon à prévenir une réaction de transligation entre le complexe actif et le chélate additionnel, qui aurait notamment pour effet de consommer totalement ledit ligand additionnel, qui ne pourrait dès lors plus piéger le lanthanide relargué. Ce risque de consommation de l'agent chélatant additionnel par transligation est plus prononcé lorsqu'il est ajouté sous forme libre que sous forme d'un complexe de calcium par exemple.

Ainsi, dans les deux stratégies décrites ci-dessus, il importe que le complexe actif soit le plus stable possible.

Or, les complexes de ligands chélateurs dérivés de PCTA comprenant une structure de type pyclène décrits dans le document EP 1 931 673, tout en ayant une bonne stabilité cinétique, ont, en général, une constante thermodynamique plus faible que celle des complexes des autres macrocycles dérivés du cyclène.

C'est notamment le cas du complexe de formule (II) représentée ci-après :

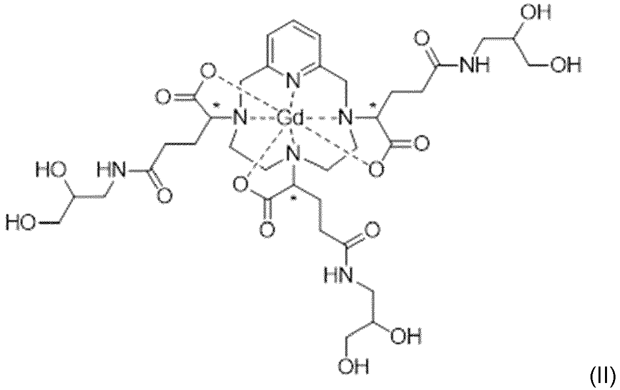

En effet, comme cela est notamment décrit dans le document WO 2014/174120, la constante d'équilibre thermodynamique correspondant à la réaction de formation du complexe de formule (II), également appelée constante de stabilité, est de 10^{14,9} (i.e. log (Kₜₕₑᵣₘ) = 14,9). A titre de comparaison, la constante de stabilité du complexe de gadolinium de l'acide 1, 4, 7, 10 tétra-azacyclododécane N, N', N", N'" tétra-acétique (DOTA-Gd), est de 10^{25,6} (i.e. log (Kₜₕₑᵣₘ) = 25,6).

Il convient toutefois de noter que le complexe de formule (II) correspond à plusieurs stéréoisomères, notamment du fait de la présence des trois atomes de carbones asymétriques situés en position α sur les chaînes latérales du complexe, par rapport aux atomes d'azote du macrocycle sur lesquels sont greffées lesdites chaînes latérales. Ces trois carbones asymétriques sont marqués d'un astérisque (*) dans la formule (II) représentée ci-dessus.

Ainsi, la synthèse du complexe de formule (II) telle que décrite dans le document EP 1 931 673 aboutit à l'obtention d'un mélange de stéréoisomères.

Les groupements aminopropanediol des chaînes latérales du complexe de formule (II) comportent également un carbone asymétrique. Ainsi, le complexe de formule (II) comprend au total 6 carbones asymétriques, et existe par conséquent sous la forme de 64 stéréoisomères de configuration. Toutefois, dans la suite de la description, la seule source de stéréoisomérie considérée pour une chaîne latérale donnée sera, par souci de simplification, celle correspondant au carbone asymétrique portant le groupement carboxylate, marqué d'un astérisque (*) dans la formule (II) représentée ci-dessus.

Dans la mesure où chacun de ces 3 carbones asymétriques peut être de configuration absolue R ou S, le complexe de formule (II) existe sous la forme de 8 familles de stéréoisomères, dénommées ci-après II-RRR, II-SSS, II-RRS, II-SSR, II-RSS, II-SRR, II-RSR et II-SRS. Plus précisément, selon la nomenclature usuelle en stéréochimie, le complexe de formule (II) existe sous la forme de 8 familles de diastéréoisomères.

L'emploi du terme « famille » se justifie en cela que chacune de ces familles regroupe plusieurs stéréoisomères, notamment du fait de la présence d'un carbone asymétrique au sein du groupement aminopropanediol, comme évoqué précédemment.

Néanmoins, dans la mesure où, dans la suite de la description, la stéréoisomérie liée au carbone asymétrique d'un groupement aminopropanediol donné ne sera pas considérée, on parlera indifféremment d'isomères, stéréoisomères ou encore diastéréoisomères II-RRR, II-SSS, II-RRS, II-SSR, II-RSS, II-SRR, II-RSR et II-SRS, sans préciser que chacun correspond à une famille de stéréoisomères.

Les inventeurs sont parvenus à séparer et à identifier par chromatographie en phase liquide à haute performance (CLHP, plus communément désignée par l'acronyme anglais HPLC) et par chromatographie en phase liquide à ultra haute performance (CLUHP, plus communément désignée par l'acronyme anglais UHPLC) 4 massifs ou groupes d'isomères du complexe de formule (II) obtenu selon le procédé de l'art antérieur, correspondant à 4 pics d'élution différents caractérisés par leur temps de rétention sur le chromatogramme, que l'on nommera iso1, iso2, iso3 et iso4 dans la suite de la description. En mettant en oeuvre le procédé décrit dans le document EP 1 931 673, les teneurs respectives des groupes iso1, iso2, iso3 et iso4 dans le mélange obtenu sont les suivantes : 20%, 20%, 40% et 20%.

Ils ont alors découvert que ces différents groupes d'isomères présentaient des propriétés physico-chimiques distinctes, et ont déterminé que le groupe d'isomères dénommé iso4, qui comprend un mélange des isomères II-RRR et II-SSS de formules (II-RRR) et (II-SSS) représentées ci-après, s'avère être le plus intéressant en tant qu'agent de contraste pour l'imagerie médicale.

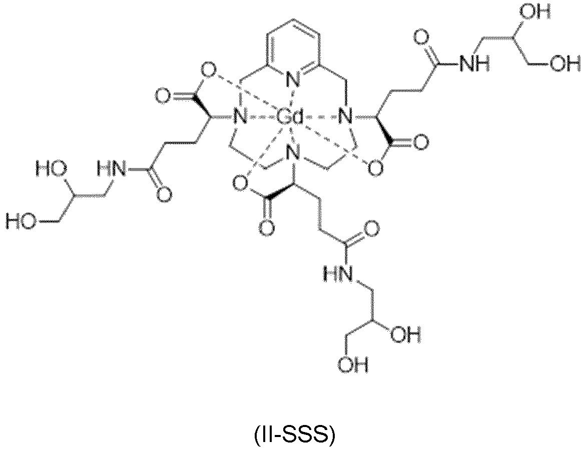

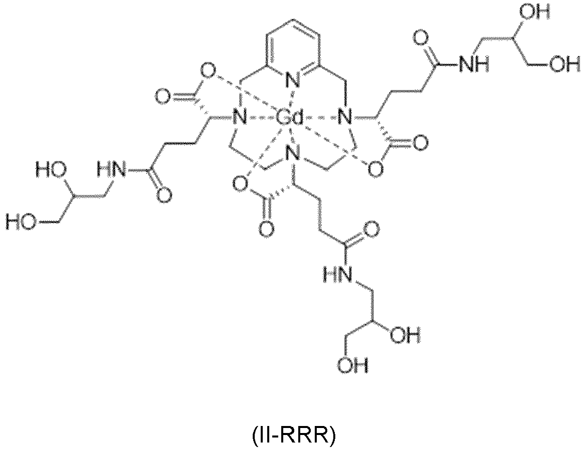

Ainsi, l'iso4 se distingue, de façon étonnante, par une stabilité thermodynamique nettement supérieure à celle du mélange de diastéréoisomères sous la forme duquel est obtenu le complexe de formule (II) en mettant en oeuvre le procédé décrit dans le document EP 1 931 673. En effet, sa constante thermodynamique d'équilibre Kt_{herm iso4} est égale à 10^{18,7} (i.e. log (K_{therm iso4}) = 18,7) cette valeur ayant été déterminée en mettant en oeuvre la méthode dans Pierrard et al., Contrast Media Mol. Imaging, 2008, 3, 243-252 et Moreau et al., Dalton Trans., 2007, 1611-1620.

De surcroît, l'iso4 est le groupe d'isomères qui présente la meilleure inertie cinétique (également appelée stabilité cinétique) parmi les quatre groupes isolés par les inventeurs. En effet, les inventeurs ont évalué l'inertie cinétique des quatre groupes d'isomères en étudiant leur cinétique de décomplexation en solution aqueuse acide (pH = 1,2), à 37°C. Les valeurs des temps de demi-vie (T_{1/2}) qui ont été déterminées pour chacun des groupes d'isomères sont indiquées dans le tableau 1 ci-dessous, le temps de demi-vie correspondant à la durée au bout de laquelle 50 % de la quantité de complexe initialement présente a été dissociée, selon la réaction de décomplexation suivante (équation 2) :

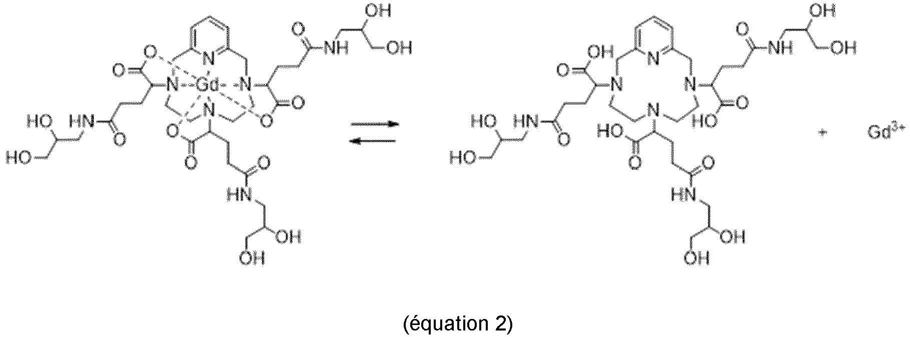

**Tableau 1 : cinétique de décomplexation des groupes d'isomères iso1 à iso4**

| **Groupes d'isomères** | **T_{1/2} (pH 1,2** - **37 °C)** |
|---|---|
| Iso1 | 18 heures |
| Iso2 | 6 heures |
| Iso3 | 8 jours |
| Iso4 | 27 jours |

A titre de comparaison, le gadobutrol ou le gadoterate, complexes macrocycliques de gadolinium, présentent respectivement une inertie cinétique de 18 heures et de 4 jours dans les mêmes conditions, tandis que les complexes linéaires de gadolinium comme le gadodiamide ou le gadopentetate se dissocient instantanément.

En outre, l'iso4 est plus stable d'un point de vue chimique que l'iso3 notamment. Les fonctions amides du complexe de formule (II) sont en effet susceptibles d'être hydrolysées. La réaction d'hydrolyse d'une fonction amide (équation 3) aboutit à la formation d'une impureté dicouplée, qui s'accompagne de la libération de 3-amino-1,2-propanediol. Les inventeurs ont étudié la cinétique de la réaction d'hydrolyse du complexe de formule (II) en solution aqueuse à pH 13, et observé que les fonctions amides de l'iso4 sont plus stables vis-à-vis de l'hydrolyse que celles de l'iso3.

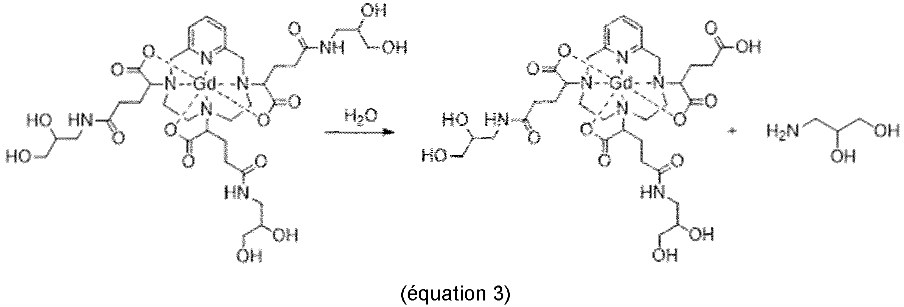

En ce qui concerne la relaxivité des différents groupes d'isomères, c'est-à-dire leur efficacité en tant qu'agent de contraste, les mesures réalisées mettent en évidence un pouvoir contrastant relativement équivalent pour les groupes iso1, iso2 et iso4, et une efficacité moindre pour l'iso3 (voir tableau 2).

**Tableau 2 : relaxivité des groupes d'isomères iso1 à iso4 à 37°C**

| **Groupes d'isomères** | **r1 20 MHz** (mM⁻¹.s⁻¹) | **r1 60 MHz** (mM⁻¹.s⁻¹) |
|---|---|---|
| Iso1 | 12.6 | 12.5 |
| Iso2 | 13.3 | 12.9 |
| Iso3 | 8.0 | 8.1 |
| Iso4 | 12.9 | 13.0 |

Les inventeurs sont parvenus à développer un nouveau procédé de préparation et de purification du complexe de formule (II) permettant d'obtenir préférentiellement les diastéréoisomères I I-RRR et I I-SSS dudit complexe, qui présentent des propriétés physico-chimiques particulièrement avantageuses. Ce procédé comprend une étape d'enrichissement isomérique, par conversion des stéréoisomères les moins stables vers les stéréoisomères les plus stables, qui, de manière surprenante, tout en étant réalisée sur le complexe intermédiaire hexaacide et non pas sur le complexe final, permet d'obtenir très majoritairement les isomères du complexe de formule (II) les plus stables.

La mise en oeuvre d'un procédé qui permet d'obtenir majoritairement les diastéréoisomères d'intérêt est incontestablement avantageuse par rapport à l'alternative consistant à préparer le mélange de stéréoisomères, pour ensuite tenter de séparer les diastéréoisomères selon les techniques usuelles et ainsi isoler les isomères d'intérêt en utilisant n'importe quelle technique de séparation bien connue dans l'art. En effet, outre le fait qu'il est plus aisé de mettre en oeuvre un procédé dépourvu d'une étape de séparation de diastéréoisomères à l'échelle industrielle, l'absence de séparation permet d'une part un gain de temps considérable, et, d'autre part, d'améliorer le rendement global du procédé, en limitant autant que possible l'obtention des diastéréosiomères non désirés qui seraient *in fine* éliminés. Par ailleurs, les techniques de séparation usuelles impliquent de manière générale une utilisation abondante de solvants, qui, au-delà du coût financier, n'est pas souhaitable pour des raisons environnementales. De surcroît, la chromatographie sur silice en particulier est à éviter, compte tenu des risques pour la santé inhérents à une exposition professionnelle à la silice, classée comme cancérogène pour l'homme (groupe 1) par le Centre International de Recherche contre le Cancer.

Comme indiqué précédemment, le procédé de préparation du complexe de formule (II) mis au point par les inventeurs repose sur une étape d'enrichissement isomérique du complexe de gadolinium d'hexaacide intermédiaire de formule (I) représentée ci-après :

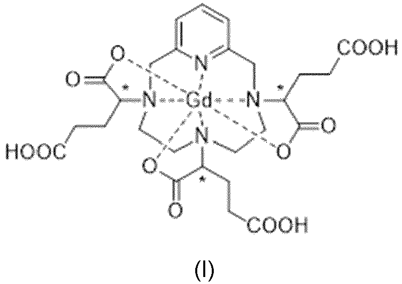

Le complexe de formule (I) correspond à plusieurs stéréoisomères, du fait de la présence des trois atomes de carbones asymétriques situés en position α sur les chaînes latérales du complexe, par rapport aux atomes d'azote du macrocycle sur lesquels sont greffées lesdites chaînes latérales. Ces trois carbones asymétriques sont marqués d'un astérisque (*) dans la formule (I) représentée ci-dessus.

Dans la mesure où chacun des 3 carbones asymétriques portant une fonction carboxylate peut être de configuration absolue R ou S, le complexe de formule (I) existe sous la forme de 8 stéréoisomères, dénommés ci-après I-RRR, I-SSS, I-RRS, I-SSR, I-RSS, I-SRR, I-RSR et I-SRS. Plus précisément, selon la nomenclature usuelle en stéréochimie, le complexe de formule (I) existe sous la forme de 4 paires d'énantiomères, diastéréoisomères entre elles.

Les inventeurs sont parvenus à séparer et à identifier par chromatographie en phase liquide à haute performance (CLHP, plus communément désignée par l'acronyme anglais HPLC) et par chromatographie en phase liquide à ultra haute performance (CLUHP, plus communément désignée par l'acronyme anglais UHPLC) 4 massifs ou groupes d'isomères du complexe de formule (I) obtenu selon le procédé décrit dans le document EP 1 931 673, correspondant à 4 pics d'élution différents caractérisés par leur temps de rétention sur le chromatogramme, que l'on nommera isoA, isoB, isoC et isoD dans la suite de la description.

L'isoD cristallise dans l'eau. Des analyses par diffraction des rayons X ont permis aux inventeurs de déterminer la structure cristalline de ce groupe d'isomères, et ainsi de découvrir qu'il comprend les diastéréoisomères I-RRR et I-SSS du complexe de formule (I), de formules (I-RRR) et (I-SSS) représentées ci-après.

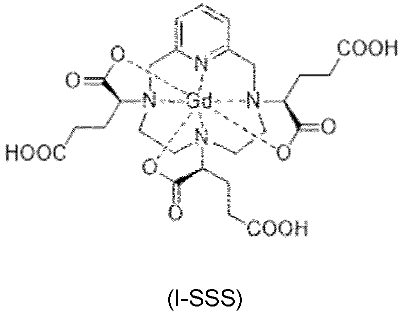

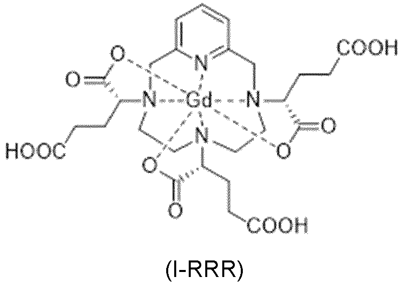

Il est à noter que les diastéréosiomères I-RRR et I-SSS du complexe de formule (I) sont énantiomères l'un de l'autre.

L'étape d'enrichissement isomérique du procédé de l'invention vise à enrichir le complexe de gadolinium d'hexaacide intermédiaire de formule (I) en isoD.

La synthèse du complexe de formule (II) implique notamment une conversion des fonctions acides carboxyliques du complexe hexaacide intermédiaire de formule (I) en fonction amide. Cette réaction d'amidification ne modifie pas la configuration absolue des trois atomes de carbones asymétriques du complexe de formule (I).

Ainsi, lorsque la réaction d'amidification est réalisée sur le complexe hexaacide de formule (I) enrichi en isoD précédemment obtenu, elle permet d'obtenir le complexe de formule (II) enrichi en iso4.

Par ailleurs, le procédé de purification développé par les inventeurs, permet, lorsqu'il est mis en oeuvre à la suite du procédé de préparation du complexe de formule (II) susmentionnée, d'obtenir le complexe de formule (II) avec un profil isomérique optimisé, mais aussi un profil d'impuretés nettement amélioré.

Ce complexe diastéréoisomériquement enrichi et purifié présentant une stabilité améliorée peut dès lors être formulé avec un ligand macrocyclique libre, tel que du DOTA libre, en lieu et place d'un complexe calcique de DOTA, dont l'emploi était recommandé dans le document WO 2014/174120. L'utilisation du DOTA libre présente notamment un avantage d'un point de vue industriel, en cela qu'il permet de supprimer une étape du procédé de synthèse de la formulation tel que décrit dans le document WO 2014/174120, à savoir l'ajout de CaCl₂.

### Composition comprenant le complexe de formule (II)

La présente invention concerne donc une composition comprenant :
un complexe de formule (II) : 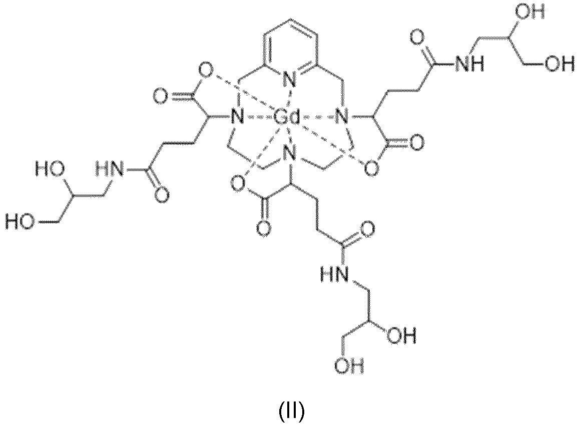 constitué d'au moins 80 % d'un excès diastéréoisomérique comprenant un mélange d'isomères II-RRR et II-SSS de formules : 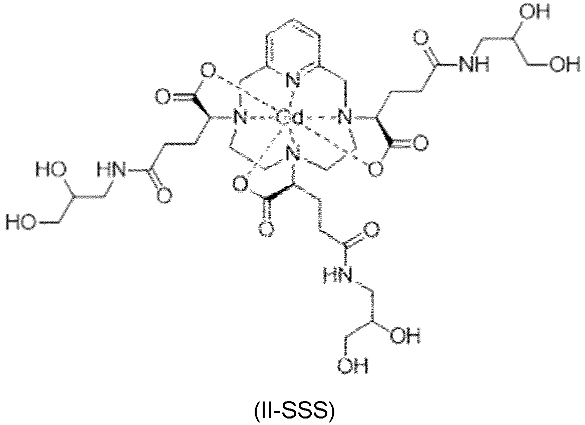 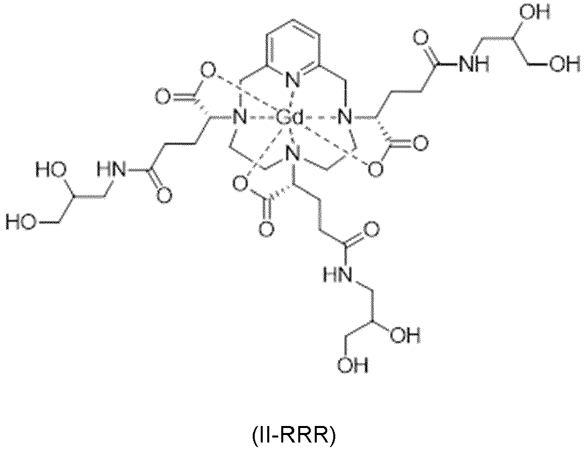 et un ligand macrocyclique libre,
la composition comprenant entre 0.002 et 0.4% mol/mol de ligand macrocyclique libre par rapport au complexe de formule (II).

Par « excès diastéréoisomérique », on entend désigner, dans le cadre de la présente invention, et en ce qui concerne le complexe de formule (II), le fait que ledit complexe est majoritairement présent sous la forme d'un isomère ou groupe d'isomères choisi(s) parmi les diastéréoisomères II-RRR, II-SSS, II-RRS, II-SSR, II-RSS, II-SRR, II-RSR et II-SRS. Ledit excès diastéréoisomérique est exprimé en pourcentage et correspond à la quantité que représente l'isomère ou le groupe d'isomères majoritaire par rapport à la quantité totale du complexe de formule (II). Il est entendu que ce pourcentage peut être aussi bien molaire que massique, dans la mesure où des isomères ont, par définition, la même masse molaire.

Dans un mode de réalisation particulier de la composition selon l'invention, le complexe de formule (II) présente au moins 85 %, notamment au moins 90 %, en particulier au moins 92 %, de préférence au moins 94 %, avantageusement au moins 97 %, plus avantageusement au moins 99 % de l'excès diastéréoisomérique comprenant le mélange d'isomères II-RRR et II-SSS.

De préférence, ledit excès diastéréoisomérique est constitué d'au moins 90 %, de préférence d'au moins 95 % du mélange d'isomères II-RRR et II-SSS.

Avantageusement, ledit excès diastéréoisomérique consiste en le mélange d'isomères II-RRR et II-SSS.

Le terme « mélange d'isomères II-RRR et II-SSS » recouvre également, par extension, le cas où seul l'un des isomères, qu'il s'agisse du II-RRR ou du II-SSS, est présent. Toutefois, le terme « mélange d'isomères II-RRR et II-SSS » désigne préférentiellement l'ensemble des cas où chacun des isomères II-RRR et II-SSS est présent en une quantité variable mais non nulle.

Dans un mode de réalisation préféré de la composition selon l'invention, les isomères II-RRR et II-SSS sont présents au sein dudit mélange dans un rapport compris entre 65/35 et 35/65, notamment entre 60/40 et 40/60, en particulier entre 55/45 et 45/55. Avantageusement, les isomères II-RRR et II-SSS sont présents au sein du mélange dans un rapport 50/50.

Plus particulièrement, l'excès diastéréoisomérique tel que défini précédemment correspond au pic 4 de la trace UHPLC (c'est-à-dire le quatrième massif d'isomères dans l'ordre d'élution et correspondant à l'iso4), caractérisé par un temps de rétention compris entre 6,0 et 6,6 minutes, typiquement d'environ 6,3 minutes, ladite trace étant obtenue en mettant en oeuvre la méthode UHPLC décrite ci-après.

Par « trace UHPLC », on entend, au sens de la présente invention, le profil des concentrations mesurées par le détecteur après passage et séparation d'un mélange de composés (en l'espèce d'isomères d'un composé) sur une phase stationnaire en fonction du temps pour une composition et un débit d'éluant donné. La trace UHPLC est constituée de différents pics ou massifs caractéristiques du composé ou du mélange de composés analysés.

### Méthode UHPLC :

- colonne CORTECS^{®} UPLC T3 150 x 2,1 mm - 1,6 µm de Waters.
   Il s'agit d'une colonne UPLC en phase inverse à particules sphériques constituées d'un noyau, préférentiellement très dur, en silice entouré d'une silice poreuse avec un greffage C18 (octadécyl) trifonctionnel, et dont les silanols ont été traités par des agents de coiffage (end-capped). Elle est en outre caractérisée par une longueur de 150 mm, un diamètre intérieur de 2,1 mm, une granulométrie de 1,6 µm, une porosité de 120 Å et un taux de carbone de 4,7 %.

De manière préférentielle, la phase stationnaire utilisée doit être compatible avec les phases mobiles aqueuses.
- conditions d'analyse :

| Echantillon | Solution aqueuse du complexe de formule (II) à 2,0 mg/mL |
|---|---|
| Température colonne | 40°C |
| Température échantillon | Température ambiante (20-25°C) |
| Débit | 0,3 mL/min |
| Volume d'injection | 1 µL |
| Détection UV | 200 nm |

- gradient phase mobile (% v /v) :

| Temps (min) | acétonitrile (100 %) | H₂SO₄ (solution aqueuse à 0,0005 % v/v) |
|---|---|---|
| 0 | 1 | 99 |
| 3 | 5 | 95 |
| 12 | 10 | 90 |

Dans la présente description, les termes « ligand macrocyclique » ou « chélate macrocyclique » peuvent être utilisés indifféremment.

Dans le cadre de la présente invention, le terme « macrocycle » désigne un cycle comportant typiquement au moins 9 atomes, qu'il s'agisse d'atomes de carbone ou d'hétéroatomes, et le « ligand macrocyclique » ou « chélate macrocyclique » est un ligand polydentate, au moins bidentate.

Par « ligand macrocyclique libre », on entend au sens de la présente invention le ligand macrocyclique sous forme libre, c'est-à-dire non complexé, en particulier à des métaux - y compris les lanthanides et les actinides - ou à des cations d'alcalino-terreux tels que le calcium ou le magnésium. En particulier, le ligand macrocyclique libre n'est pas sous forme de complexe avec le gadolinium, et n'est pas introduit dans la composition sous la forme d'un complexe faible, typiquement de calcium, sodium, zinc ou magnésium, comme décrit dans le document US 5,876,695, la présence desdits cations à l'état de trace dans la composition, et donc des complexes correspondants n'étant toutefois pas exclue.

Comme discuté précédemment, la formulation du complexe de formule (II) avec un ligand macrocyclique libre, et non un complexe faible dudit ligand macrocyclique comme recommandé dans le document EP 1 931 673, et rendue possible par la stabilité améliorée du complexe de formule (II) diastéréoisomériquement enrichi selon l'invention.

Dans un mode de réalisation avantageux, la composition selon l'invention a une concentration en gadolinium libre inférieure à 1 ppm (m/v), préférentiellement inférieure à 0,5 ppm (m/v).

Dans la présente description, sauf mention contraire, on utilise indifféremment les expressions « Gd », « gadolinium » et « Gd³⁺ » pour désigner l'ion Gd³⁺. Par extension, il peut également s'agir d'une source de gadolinium libre, tels que le chlorure de gadolinium (GdCl₃) ou l'oxyde de gadolinium (Gd₂O₃).

Dans la présente invention, on désigne par « Gd libre » les formes non complexées du gadolinium, et de préférence disponibles pour une complexation. Il s'agit typiquement de l'ion Gd³⁺ solubilisé dans l'eau. Par extension, il peut également s'agir d'une source de gadolinium libre, tels que le chlorure de gadolinium (GdCl₃) ou l'oxyde de gadolinium (Gd₂O₃).

Le gadolinium sous forme libre est typiquement mesuré par dosage colorimétrique, généralement le xylénol orange ou l'Arsenazo (III). En l'absence d'ion métallique (tel que le gadolinium), ces indicateurs ont une couleur spécifique : à pH acide, le xylenol orange a une couleur jaune, tandis que l'Arsenazo possède une couleur rose. En présence de gadolinium, leur couleur vire au violet.

La détermination visuelle du virage de la couleur de la solution permet de vérifier la présence ou l'absence de gadolinium dans la solution.

Par ailleurs, il est possible de mesurer quantitativement le gadolinium libre se trouvant dans la solution via un dosage retour, utilisant par exemple l'EDTA comme chélate « faible » du Gadolinium. Dans un tel dosage, l'indicateur coloré est ajouté jusqu'à obtention d'une couleur violette. Puis de l'EDTA, un ligand du gadolinium est ajouté au mélange au goutte à goutte. L'EDTA étant un complexant plus fort que l'indicateur coloré, le gadolinium va changer de ligand et va quitter l'indicateur coloré pour se complexer préférentiellement à l'EDTA. L'indicateur coloré va donc progressivement retrouver sa forme non complexée.

Lorsque la quantité d'EDTA ajoutée est égale à la quantité initiale de Gd libre, l'indicateur coloré est entièrement sous sa forme libre et la solution "tourne" au jaune. La quantité d'EDTA ajoutée étant connue, cela permet de connaître la quantité initiale de Gd libre dans la solution à doser.

Ces méthodes sont bien connues de l'homme du métier, et décrites notamment par document Barge et al. (Contrast Media and Molecular Imaging 1, 2006, 184-188).

Ces méthodes colorimétriques sont ainsi usuellement mises en oeuvre sur une solution dont le pH est compris entre 4 et 8. En effet, en dehors de ces plages de pH, la précision de la mesure peut se trouver affectée du fait d'une modification (voire une suppression) du virage coloré.

Ainsi, si besoin, le pH de l'échantillon à doser est ajusté pour être compris entre 4 et 8. Notamment, si le pH de l'échantillon est acide, et en particulier inférieur à 4, on ajuste avantageusement le pH par ajout d'une base, puis la mesure du Gd libre est effectuée sur l'échantillon au pH ajusté.

La composition selon l'invention présente ainsi une stabilité sur la durée, c'est-à-dire que sa composition reste conforme aux spécifications en termes de concentration de gadolinium libre (en particulier sa concentration en Gd libre reste inférieure à 1 ppm (m/v)), sur une durée d'au moins 3 ans, préférentiellement d'au moins 4 ans ou plus préférentiellement d'au moins 5 ans, notamment en termes de teneur en métal paramagnétique libre. Selon les directives ICH, une observation de cette stabilité pendant 6 mois à 40°C est considérée comme une bonne indication d'une stabilité de 3 ans à 25°C.

Dans un mode de réalisation particulier, la composition selon l'invention présente une concentration comprise entre 0,01 et 1,5 mol.L⁻¹, préférentiellement comprise entre 0,2 et 0,7 mol.L⁻¹, plus préférentiellement entre 0,3 et 0,6 mol.L⁻¹ en complexe de formule (II) décrit ci-dessus.

Le complexe de formule (II) est dosé par les méthodes connues de l'homme du métier. On peut notamment le doser après une minéralisation et un dosage du gadolinium total présent dans la composition, par spectrométrie d'émission optique (appelée aussi ICP-AES ou ICP Atomic Emission Spectrometry).

La teneur en complexe de formule (II) permet à cette composition d'avoir un pouvoir contrastant optimal tout en ayant une viscosité satisfaisante. En effet, en dessous de 0,01 mol.L⁻¹ de complexe de formule (II) décrit ci-dessus, les performances en tant que produit de contraste sont moins satisfaisantes, et à une concentration supérieure à 1,5 mol.L⁻¹, la viscosité de cette composition devient trop importante pour une manipulation aisée.

Dans un mode de réalisation particulier, la composition selon l'invention comprend entre 0,01 et 0,3 % mol/mol, de préférence entre 0,02 et 0,2 % mol/mol, plus préférentiellement entre 0,05 et 0,15 % mol/mol de ligand macrocyclique libre par rapport au complexe de formule (II).

Avantageusement, le ligand macrocyclique est sélectionné dans le groupe constitué de DOTA, NOTA, DOSA, BT-DO3A, HP-DO3A, PCTA, DOTA-GA et leurs dérivés.

De préférence, il s'agit du DOTA (acide 1,4,7,10-tétraazacyclododecane-1,4,7,10-tétraacetique).

La concentration de DOTA libre dans la composition est typiquement mesurée par un dosage retour au cuivre, en utilisant par exemple le sulfate de cuivre comme source d'ion cuivre.

Dans cette méthode, bien connue de l'homme du métier, on utilise de préférence une solution contenant une concentration initiale connue Q₀ de sulfate de cuivre, cette concentration étant supérieure à la quantité de ligand libre dans la solution. A cette solution de sulfate de cuivre est ajoutée la solution à doser, contenant du DOTA libre en quantité Q₁ à déterminer. Le DOTA est un très bon complexant du cuivre : on observe donc la formation d'un complexe DOTA-cuivre.

On effectue alors avantageusement un dosage retour du cuivre restant libre dans la solution par potentiométrie. Pour ce faire, de l'EDTA est par exemple ajouté au mélange au goutte à goutte. L'EDTA va complexer le cuivre libre en solution sans pour autant décomplexer le DOTA-cuivre, car le DOTA est un complexant plus fort que l'EDTA. Lorsque la quantité d'EDTA ajoutée Q₂ est égale à la quantité de cuivre libre en solution, on observe une chute brusque du potentiel de la solution.

Connaissant la quantité initiale Q₀ de cuivre et la quantité d'EDTA ajoutée Q₂, la soustraction de ces deux valeurs Q₀ - Q₂ donne la quantité de DOTA libre dans la solution à doser Q₁.

Alternativement, des méthodes HPLC peuvent être utilisées, notamment la méthode HILIC LC-UV.

Ces méthodes de mesure (en particulier les méthodes potentiométriques) sont mises en oeuvre sur des solutions dont le pH est avantageusement compris entre 4 et 8. Ainsi, si besoin, le pH de l'échantillon à doser est ajusté pour être compris entre 4 et 8. Notamment, si le pH de l'échantillon est acide, et en particulier inférieur à 4, on ajuste avantageusement le pH par ajout d'une base telle que la méglumine, puis la mesure du DOTA libre est effectuée sur l'échantillon au pH ajusté.

De manière préférentielle, les proportions précisées dans la présente invention et en particulier ci-dessus sont des proportions avant stérilisation de la composition.

De façon avantageuse, le pH de la composition est compris entre 4,5 et 8,5, préférentiellement entre 5 et 8, avantageusement entre 6 et 8, notamment entre 6,5 et 8. Ces gammes de pH permettent notamment de limiter l'apparition de certaines impuretés et de favoriser la complexation de l'ion de métal paramagnétique M.

En particulier, la composition selon l'invention peut être tamponnée, c'est-à-dire qu'elle peut comprendre en outre un tampon choisi parmi les tampons d'usage établis pour la gamme de pH 5 à 8 et préférentiellement parmi les tampons lactate, tartrate, malate, maléate, succinate, ascorbate, carbonate, Tris (Tris(hydroxymethyl)aminomethane), HEPES (acide 2-[4-(2-Hydroxyethyl)-1-piperazine]ethanesulfonique), MES (acide 2-morpholino éthanesulphonique) et les mélanges de ceux-ci, et préférentiellement un tampon choisi parmi les tampons Tris, lactate, tartrate, carbonate, le MES et les mélanges de ceux-ci. Avantageusement, la composition selon l'invention comprend le tampon Tris.

La composition objet de l'invention est préférentiellement stérile.

### Procédé de préparation du complexe de formule (II)

Le complexe de formule (II) d'une composition selon l'invention peut notamment être obtenu par un procédé de préparation comprenant les étapes successives suivantes :
**a)** Complexation de l'hexaacide de formule (III) suivante : 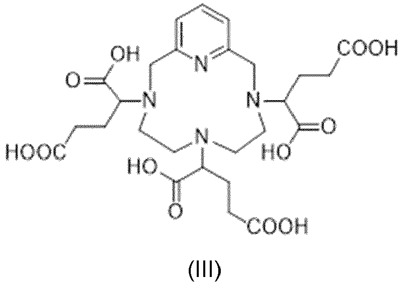 avec du gadolinium pour obtenir le complexe de gadolinium d'hexaacide de formule (I) telle que définie précédemment,
**b)** Isomérisation par chauffage du complexe de gadolinium d'hexaacide de formule (I) dans une solution aqueuse à pH compris entre 2 et 4, pour obtenir un complexe diastéréoisomériquement enrichi constitué d'au moins 80 % d'un excès diastéréoisomérique comprenant un mélange des isomères I-RRR et I-SSS dudit complexe de gadolinium d'hexaacide de formule (I), et
**c)** Formation, à partir du complexe diastéréoisomériquement enrichi obtenu à l'étape b), du complexe de formule (II), par réaction avec le 3-amino-1,2-propanediol.

Ce procédé de préparation n'est pas couvert par le texte des revendications. Sa description détaillée ci-après est néanmoins considérée comme utile à la compréhension de l'invention.

### ▪ Etape a)

Au cours de cette étape a lieu une réaction de complexation entre l'hexaacide de formule (III) et le gadolinium, qui permet d'obtenir le complexe de gadolinium d'hexaacide de formule (I) telle que définie précédemment.

Selon un mode de réalisation particulier du procédé permettant de préparer le complexe de formule (II) d'une composition selon l'invention, l'étape a) comprend la réaction entre l'hexaacide de formule (III) et une source de Gd libre dans de l'eau.

Dans un mode de réalisation préféré du procédé non revendiqué, la source de Gd libre est du GdCl₃ ou du Gd₂O₃, de préférence du Gd₂O₃.

De préférence, les réactifs utilisés à l'étape a), c'est-à-dire la source de gadolinium (typiquement l'oxyde de gadolinium), l'hexaacide de formule (III) et l'eau, sont les plus purs possibles, notamment en ce qui concerne les impuretés métalliques.

Ainsi, la source de gadolinium sera avantageusement l'oxyde de gadolinium, de préférence avec une pureté supérieure à 99,99 %, et de manière encore préférée supérieure à 99,999 %.

L'eau utilisée dans le procédé comprend de préférence moins de 50 ppm de calcium, de manière encore préférée moins de 20 ppm, et de manière préférée entre toutes moins de 15 ppm de calcium. Généralement, l'eau employée dans le procédé est de l'eau désionisée, de l'eau pour injection (eau ppi) ou de l'eau purifiée.

Avantageusement, les quantités des réactifs (l'hexaacide de formule (III) et le gadolinium) utilisées lors de cette étape a) correspondent aux, ou sont proches des, proportions stoechiométriques, telles que dictées par l'équation-bilan de la réaction de complexation ayant lieu au cours de cette étape.

Par « proche des proportions stoechiométriques », on entend signifier que l'écart entre les proportions molaires dans lesquelles sont introduits les réactifs et les proportions stoechiométriques est inférieur à 15 %, notamment inférieur à 10 %, de préférence inférieur à 8 %.

Le gadolinium peut notamment être introduit en léger excès par rapport aux proportions stoechiométriques. Le rapport de la quantité de matière introduite en gadolinium sur la quantité de matière introduite en hexaacide de formule (III) est alors supérieur à 1, mais typiquement inférieur à 1,15, notamment inférieur à 1,10, avantageusement inférieur à 1,08. Autrement dit, la quantité de gadolinium introduite est supérieure à 1 équivalent (éq.), mais typiquement inférieure à 1,15 éq., notamment inférieure à 1,10 éq., avantageusement inférieure à 1,08 éq., par rapport à la quantité d'hexaacide de formule (III) introduite, qui correspond quant à elle à 1 équivalent. Dans le mode de réalisation préféré selon lequel la source de gadolinium libre est Gd₂O₃, la quantité de Gd₂O₃ introduite est alors typiquement supérieure à 0,5 éq., mais inférieure à 0,575 éq., notamment inférieure à 0,55 éq., avantageusement inférieure à 0,54 éq., par rapport à la quantité d'hexaacide de formule (III) introduite (1 éq.).

Selon un mode de réalisation particulier du procédé non revendiqué, l'étape a) comprend les étapes successives suivantes :
**a1)** Préparation d'une solution aqueuse d'hexaacide de formule (III), et
**a2)** Ajout, à la solution aqueuse obtenue à l'étape a1), d'une source de gadolinium libre.

Dans ce mode de réalisation, la teneur en hexaacide de formule (III) dans la solution aqueuse préparée lors de l'étape a1) est typiquement comprise entre 10 % et 60 %, notamment entre 15 % et 45 %, de préférence entre 20 % et 35 %, avantageusement entre 25 % et 35 %, de manière encore plus avantageuse entre 25 % et 30 % en poids par rapport au poids total de la solution aqueuse.

De manière préférentielle, les étapes a) et b) sont effectuées selon un mode de réalisation monotope (ou « one-pot » en anglais), c'est-à-dire dans le même réacteur et sans étape intermédiaire d'isolement ou de purification.

Ainsi, dans ce mode de réalisation préféré, le complexe de gadolinium d'hexaacide de formule (I) formé au cours de l'étape a) est directement soumis à l'étape b) d'isomérisation, sans être isolé ou purifié, et dans le même réacteur que celui utilisé pour l'étape a).

### ▪ Etape b)

Le complexe de gadolinium d'hexaacide de formule (I) formé par la réaction de complexation entre l'hexaacide de formule (III) et le gadolinium au cours de l'étape a) est initialement obtenu sous la forme d'un mélange de diastéréoisomères.

L'étape b) vise à enrichir le mélange de diastéréoisomères en les isomères I-RRR et I-SSS, pour obtenir le complexe de gadolinium d'hexaacide de formule (I) diastéréoisomériquement enrichi constitué d'au moins 85 %, notamment d'au moins 90 %, en particulier d'au moins 95 %, de préférence d'au moins 97 %, avantageusement d'au moins 98 %, plus avantageusement d'au moins 99 % d'un excès diastéréoisomérique comprenant le mélange des isomères I-RRR et I-SSS.

Par « excès diastéréoisomérique » on entend désigner, dans le cadre de la présente invention, et en ce qui concerne le complexe de gadolinium d'hexaacide de formule (I), le fait que ledit complexe est majoritairement présent sous la forme d'un isomère ou groupe d'isomères choisi(s) parmi les diastéréoisomères I-RRR, I-SSS, I-RRS, I-SSR, I-RSS, I-SRR, I-RSR et I-SRS. Ledit excès diastéréoisomérique est exprimé en pourcentage, et correspond à la quantité que représente l'isomère ou le groupe d'isomères majoritaire par rapport à la quantité totale du complexe de gadolinium d'hexaacide de formule (I). Il est entendu que ce pourcentage peut être aussi bien molaire que massique, dans la mesure où des isomères ont, par définition, la même masse molaire.

De préférence, ledit excès diastéréoisomérique est constitué d'au moins 90 %, de préférence d'au moins 95 % du mélange d'isomères I-RRR et I-SSS.

Avantageusement, ledit excès diastéréoisomérique consiste en le mélange d'isomères I-RRR et I-SSS.

Les inventeurs ont en effet découvert que des facteurs tels que le pH et la température de la solution de complexe de gadolinium d'hexaacide de formule (I) obtenue à l'issue de l'étape a) ont une influence sur le rapport dans lequel les différents isomères du complexe de formule (I) sont présents au sein du mélange de diastéréoisomères. Au cours du temps, le mélange tend à s'enrichir en un groupe d'isomères comprenant les isomères qui sont, de façon étonnante, les plus stables thermodynamiquement mais aussi chimiquement, en l'espèce les isomères I-RRR et I-SSS.

Le terme « mélange d'isomères I-RRR et I-SSS » recouvre également, par extension, le cas où seul l'un des isomères, qu'il s'agisse du I-RRR ou du I-SSS, est présent.

Toutefois, dans un mode de réalisation préféré du procédé non revendiqué, les isomères I-RRR et I-SSS sont présents au sein dudit mélange dans un rapport compris entre 65/35 et 35/65, notamment entre 60/40 et 40/60, en particulier entre 55/45 et 45/55. Avantageusement, le mélange de d'isomères I-RRR/I-SSS est un mélange racémique (50/50).

L'étape b) d'isomérisation du complexe de gadolinium d'hexaacide de formule (I) dans une solution aqueuse est typiquement conduite à un pH compris entre 2 et 4, notamment entre 2 et 3, avantageusement entre 2,2 et 2,8.

Le pH est préférentiellement ajusté avec un acide, de préférence un acide inorganique, tel que l'acide chlorhydrique, l'acide bromhydrique, l'acide sulfurique, l'acide nitrique ou l'acide phosphorique, par exemple avec de l'acide chlorhydrique.

Il est tout à fait surprenant que dans de telles conditions de pH, un enrichissement du mélange en des isomères particuliers, en l'espèce les isomères I-RRR et I-SSS, se produise, dans la mesure où il est connu dans l'art que les chélates de gadolinium sont caractérisés par une faible inertie cinétique en milieu acide. En effet, plus la concentration en ions H⁺ est élevée dans le milieu, plus la probabilité qu'un proton soit transféré sur l'un des atomes donneurs du ligand est forte, entrainant ainsi la dissociation du complexe. Par conséquent, l'homme du métier se serait attendu à ce que le fait de placer le complexe de gadolinium d'hexaacide de formule (I) en une solution aqueuse à un pH compris entre 2 et 4 entraîne la dissociation dudit complexe, et non pas son isomérisation en I-RRR et I-SSS.

Il est à noter que la gamme de pH recommandée par le document EP 1 931 673 pour la complexation de l'hexaacide de formule (III), à savoir 5,0 - 6,5, ne permet pas d'obtenir le complexe de formule (I) enrichi en ses isomères I-RRR et I-SSS.

L'étape b) est réalisée typiquement à une température comprise entre 80°C et 130°C, notamment entre 90°C et 125°C, de préférence entre 98°C et 122°C, avantageusement entre 100°C et 120°C, typiquement pendant une durée comprise entre 10h et 72h, notamment entre 10h et 60h, avantageusement entre 12h et 48h.

Contre toute attente, de telles conditions de température, qui, cumulées aux conditions de pH susmentionnées, devraient favoriser l'instabilité du chélate de gadolinium, n'aboutissent pas à sa décomplexation ou à la formation de toute autre impureté, mais à son isomérisation en I-RRR et I-SSS.

Dans un mode de réalisation particulier du procédé non revendiqué, la solution aqueuse de l'étape b) comprend de l'acide acétique. L'étape b) est alors avantageusement réalisée à une température comprise entre 100°C et 120°C, notamment entre 110°C et 118°C, typiquement pendant une durée comprise entre 12h et 48h, notamment entre 20h et 30h, en particulier entre 24h et 26h.

L'acide acétique est de préférence ajouté avant le chauffage de la solution de complexe de gadolinium d'hexaacide de formule (I) obtenue lors de l'étape a) dans une quantité telle que la teneur en acide acétique est comprise entre 25 % et 75 %, notamment entre 40 % et 50 % en masse par rapport à la masse d'hexaacide de formule (III) utilisée lors de l'étape a).

Lorsque la solution aqueuse est chauffée à une température avantageusement comprise entre 100°C et 120°C, typiquement entre 110°C et 118°C, de l'acide acétique est ajouté au fur et à mesure que l'eau s'évapore, de façon à maintenir un volume de solution constant.

Selon un mode de réalisation préféré du procédé non revendiqué, à l'issue de l'étape b), le complexe diastéréoisomériquement enrichi est isolé par cristallisation, de préférence par cristallisation par ensemencement.

Dans ce mode de réalisation, l'étape b) comprend les étapes successives suivantes :
b1) Isomérisation par chauffage du complexe de gadolinium d'hexaacide de formule (I) dans une solution aqueuse à pH compris entre 2 et 4, pour obtenir un complexe diastéréoisomériquement enrichi constitué d'au moins 80 % de l'excès diastéréoisomérique comprenant le mélange des isomères I-RRR et I-SSS dudit complexe de gadolinium d'hexaacide de formule (I), et
b2) Isolement par cristallisation dudit complexe diastéréoisomériquement enrichi, de préférence par cristallisation par ensemencement.

L'étape b2) de cristallisation vise d'une part à éliminer les impuretés éventuellement présentes dans la solution aqueuse, qui peuvent résulter d'étapes antérieures, de façon à obtenir un produit de plus grande pureté, décoloré, sous forme de cristaux, et d'autre part à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b1). En effet, les isomères I-RRR et I-SSS du complexe hexaacide de formule (I) cristallisent dans l'eau. En revanche, le complexe de gadolinium d'hexaacide de formule (I) non enrichi en lesdits isomères ne cristallise pas.

Le fait que les isomères I-RRR et I-SSS, en lesquels le complexe tend à s'enrichir au cours de l'étape b) (et ce contre toute attente, au vu des conditions dans lesquelles elle est réalisée), sont les seuls isomères du complexe à cristalliser dans l'eau constitue un résultat tout à fait inattendu. L'isomérisation et la cristallisation contribuent ainsi de manière synergique à l'enrichissement en isomères I-RRR et I-SSS, et dès lors à l'efficacité globale du procédé selon l'invention.

Par ailleurs, il est à noter que la cristallisation dans l'eau des isomères d'intérêt du complexe de gadolinium d'hexaacide de formule (I) permet d'éviter un ajout de solvant tel que décrit dans l'exemple 7 du document EP 1 931 673, qui met en jeu une étape de précipitation dans l'éthanol du sel trisodique dudit complexe.

L'étape b2) est réalisée avantageusement à une température comprise entre 10°C et 70°C, notamment entre 30°C et 65°C, en particulier entre 35°C et 60°C.

Selon une variante, après abaissement de la température de la solution aqueuse, de façon à ce que celle-ci soit comprise dans les gammes indiquées ci-dessus, le processus de cristallisation est induit par ensemencement. La « cristallisation par ensemencement », aussi appelée « cristallisation par amorçage », comprend l'introduction dans le réacteur dans lequel la cristallisation est effectuée (aussi appelé cristallisoir) d'une quantité connue de cristaux, appelée « semence » ou « amorce ». Cela permet de diminuer le temps de cristallisation. La cristallisation par ensemencement est bien connue de l'homme du métier. Dans le procédé, l'ensemencement par utilisation d'une amorce, en l'espèce des cristaux de complexe de gadolinium d'hexaacide de formule (I) diastéréoisomériquement enrichi ajoutés dans la solution aqueuse du complexe diastéréoisomériquement enrichi dont la température a été préalablement abaissée, permet d'obtenir une nucléation, et ainsi d'initier la cristallisation. La durée de la cristallisation par ensemencement est avantageusement comprise entre 2h et 20h, de préférence entre 6h et 18h, typiquement, elle est de 16h.

Les cristaux de complexe de gadolinium d'hexaacide de formule (I) diastéréoisomériquement enrichi sont alors isolés typiquement par filtration et séchage, en mettant en oeuvre toute technique bien connue de l'homme de l'art.

Avantageusement, le degré de pureté du complexe de gadolinium d'hexaacide de formule (I) diastéréoisomériquement enrichi isolé à l'issue de l'étape b2) est supérieur à 95 %, notamment supérieur à 98 %, avantageusement supérieur à 99 %, ledit degré de pureté étant exprimé en pourcentage massique du complexe de formule (I) par rapport à la masse totale obtenue à l'issue de l'étape b2).

Dans un mode de réalisation particulier du procédé non revendiqué, le complexe diastéréoisomériquement enrichi de l'étape b) isolé par cristallisation est à nouveau purifié par recristallisation, pour obtenir un complexe diastéréoisomériquement enrichi et purifié.

Dans ce mode de réalisation, l'étape b) comprend, outre les étapes successives b1) et b2) précédemment décrites, une étape **b3)** de purification par recristallisation du complexe de gadolinium d'hexaacide de formule (I) diastéréoisomériquement enrichi isolé.

L'étape b3) de recristallisation vise, à l'instar de l'étape b2) de cristallisation, d'une part, à obtenir un produit de plus grande pureté, et, d'autre part, à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b2).

L'étape b3) comprend typiquement les sous-étapes successives suivantes :
- mise en suspension du complexe de gadolinium d'hexaacide de formule (I) diastéréoisomériquement enrichi isolé lors de l'étape b2) en solution aqueuse, de préférence dans de l'eau,
- solubilisation dudit complexe par chauffage à une température avantageusement comprise entre 80°C et 120°C, par exemple à 100°C,
- recristallisation, de préférence par ensemencement, à une température avantageusement comprise entre 10°C et 90°C, notamment entre 20°C et 87°C, en particulier entre 55°C et 85°C, typiquement pendant une durée comprise entre 2h et 20h, notamment entre 6h et 18h, et
- isolement des cristaux de complexe de gadolinium d'hexaacide de formule (I) diastéréoisomériquement enrichi et purifié, par exemple par filtration et séchage.

Le degré de pureté du complexe de gadolinium d'hexaacide de formule (I) diastéréoisomériquement enrichi purifié isolé à l'issue de l'étape b3) est typiquement supérieur à 98 %, notamment supérieur à 99 %, avantageusement supérieur à 99,5 %, ledit degré de pureté étant exprimé en pourcentage massique du complexe de formule (I) par rapport à la masse totale obtenue à l'issue de l'étape b2).

Dans un autre mode de réalisation du procédé non revendiqué, le complexe diastéréoisomériquement enrichi de l'étape b) est encore enrichi par décomplexation sélective des diastéréoisomères du complexe de formule (I) autres que les diastéréoisomères I-RRR et I-SSS, *i.e.* par décomplexation sélective des diastéréoisomères I-RSS, I-SRR, I-RSR, I-SRS, I-RRS et I-SSR.

Dans ce mode de réalisation, l'étape b) comprend, outre les étapes successives b1) et b2) précédemment décrites, une étape **b4)** de décomplexation sélective des diastéréoisomères du complexe de formule (I) autres que les diastéréoisomères I-RRR et I-SSS. Dans cette variante, l'étape b) peut en outre comprendre l'étape b3) précédemment décrite, ladite étape b3) étant mise en oeuvre entre les étapes b2) et b4), ou après l'étape b4).

L'étape b4) de décomplexation sélective vise à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b2) ou à l'issue de l'étape b3), lorsque celle-ci est mise en oeuvre préalablement à l'étape b4).

L'étape b4) comprend typiquement les sous-étapes successives suivantes :
- mise en suspension du complexe de gadolinium d'hexaacide de formule (I) diastéréoisomériquement enrichi isolé lors de l'étape b2) ou lors de l'étape b3) dans de l'eau,
- ajout d'une base, par exemple de soude,
- chauffage à une température avantageusement comprise entre 30°C et 60°C, notamment entre 35°C et 55°C, par exemple à 40°C, typiquement pendant une durée comprise entre 2h et 20h, notamment entre 10h et 18h,
- refroidissement à une température avantageusement comprise entre 10°C et 30°C, par exemple à 30°C, et
- isolement du complexe de gadolinium d'hexaacide de formule (I) diastéréoisomériquement enrichi et purifié, par exemple par filtration et séchage.

L'étape b4) est rendue possible par le fait que les isomères I-RRR et I-SSS sont les plus stables en milieu basique. De telles conditions basiques favorisent la formation d'hydroxyde de gadolinium, et par conséquent la décomplexation des isomères les moins stables. Ainsi, il convient de noter que, de manière surprenante, les isomères I-RRR et I-SSS sont plus stables à la fois en milieu acide, ce qui permet l'étape b1) d'isomérisation, et en milieu basique, ce qui permet l'étape b4) de décomplexation sélective.

Dans un mode de réalisation préféré du procédé non revendiqué, le complexe diastéréoisomériquement enrichi obtenu à l'issue de l'étape b) selon l'une quelconque des variantes décrites ci-dessus présente au moins 85 %, notamment au moins 90 %, en particulier au moins 95 %, de préférence au moins 97 %, avantageusement au moins 98 %, plus avantageusement au moins 99 % de l'excès diastéréoisomérique comprenant le mélange des isomères I-RRR et I-SSS.

De préférence, ledit excès diastéréoisomérique est constitué d'au moins 90 %, de préférence d'au moins 95 % du mélange d'isomères I-RRR et I-SSS.

Avantageusement, ledit excès diastéréoisomérique consiste en le mélange d'isomères I-RRR et I-SSS.

Le terme « mélange d'isomères I-RRR et I-SSS » recouvre également, par extension, le cas où seul l'un des isomères, qu'il s'agisse du I-RRR ou du I-SSS, est présent. Toutefois, le terme « mélange d'isomères I-RRR et I-SSS » désigne préférentiellement l'ensemble des cas où chacun des isomères I-RRR et I-SSS est présent en une quantité variable mais non nulle.

Dans un mode de réalisation préféré du procédé non revendiqué, les isomères I-RRR et I-SSS sont présents au sein dudit mélange dans un rapport compris entre 65/35 et 35/65, notamment entre 60/40 et 40/60, en particulier entre 55/45 et 45/55. Avantageusement, le mélange de d'isomères I-RRR/I-SSS est un mélange racémique (50/50).

### ▪ Etape c)

L'étape c) vise à former le complexe de formule (II) à partir de son précurseur, le complexe de gadolinium d'hexaacide de formule (I) diastéréoisomériquement enrichi obtenu lors de l'étape b).

Au cours de cette étape, les trois fonctions acides carboxyliques du complexe hexaacide de formule (I) portées par les atomes de carbone situés en position γ sur les chaînes latérales du complexe, par rapport aux atomes d'azote du macrocycle sur lesquels sont greffées lesdites chaînes latérales, sont converties en fonctions amide, par réaction d'amidification avec le 3-amino-1,2-propanediol, sous forme racémique ou énantiomériquement pure, de préférence sous forme racémique.

Cette réaction d'amidification ne modifie pas la configuration absolue des trois atomes de carbones asymétriques situés en position α sur les chaînes latérales, par rapport aux atomes d'azote du macrocycle sur lesquels sont greffées lesdites chaînes latérales. Par conséquent, l'étape c) permet d'obtenir le complexe de formule (II) avec un excès diastéréoisomérique comprenant un mélange des isomères II-RRR et II-SSS identique à l'excès diastéréoisomérique comprenant un mélange des isomères I-RRR et I-SSS avec lequel est obtenu le complexe de gadolinium d'hexaacide de formule (I) diastéréoisomériquement enrichi obtenu à l'issue de l'étape b), qui est d'au moins 80 %.

Dans un mode de réalisation préféré du procédé non revendiqué, le complexe de formule (II) obtenu à l'issue de l'étape c) présente au moins 85 %, notamment au moins 90 %, en particulier au moins 92 %, de préférence au moins 94 %, avantageusement au moins 97 %, plus avantageusement au moins 99 % de l'excès diastéréoisomérique comprenant le mélange d'isomères II-RRR et II-SSS.

De préférence, ledit excès diastéréoisomérique est constitué d'au moins 90 %, de préférence d'au moins 95 % du mélange d'isomères II-RRR et II-SSS.

Avantageusement, ledit excès diastéréoisomérique consiste en le mélange d'isomères II-RRR et II-SSS.

Le terme « mélange d'isomères II-RRR et II-SSS » recouvre également, par extension, le cas où seul l'un des isomères, qu'il s'agisse du II-RRR ou du II-SSS, est présent. Toutefois, le terme « mélange d'isomères II-RRR et II-SSS » désigne préférentiellement l'ensemble des cas où chacun des isomères II-RRR et II-SSS est présent en une quantité variable mais non nulle.

Dans un mode de réalisation préféré, les isomères II-RRR et II-SSS sont présents au sein dudit mélange dans un rapport compris entre 65/35 et 35/65, notamment entre 60/40 et 40/60, en particulier entre 55/45 et 45/55. Avantageusement, les isomères II-RRR et II-SSS sont présents au sein du mélange dans un rapport 50/50.

La réaction d'amidification peut être effectuée selon toutes les méthodes bien connues de l'homme du métier, notamment en présence d'un agent activant des fonctions acides carboxyliques et/ou en catalyse acide.

Elle peut notamment être réalisée selon les méthodes décrites dans le brevet EP 1 931 673, notamment au paragraphe [0027] de ce brevet.

Dans un mode de réalisation particulier du procédé non revendiqué, l'étape c) comprend l'activation des fonctions acides carboxyliques (-COOH) du complexe hexaacide de formule (I) portées par les atomes de carbone situés en position γ sur les chaînes latérales du complexe, par rapport aux atomes d'azote du macrocycle sur lesquels sont greffées lesdites chaînes latérales, sous la forme de fonctions dérivées comportant un groupement carbonyle (C=O), qui sont telles que l'atome de carbone du groupement carbonyle est plus électrophile que l'atome de carbone du groupement carbonyle des fonctions acides carboxyliques. Ainsi, selon ce mode de réalisation particulier, lesdites fonctions acides carboxyliques peuvent notamment être activées sous la forme de fonctions esters, de chlorures d'acyle, d'anhydrides d'acide, ou sous toute forme activée susceptible de conduire à une liaison amide. Les formes activées susceptibles de conduire à une liaison amide sont bien connues de l'homme du métier et peuvent être par exemple obtenues par l'ensemble des méthodes connues en chimie peptidique pour créer une liaison peptidique. Des exemples de telles méthodes sont données dans la publication Synthesis of peptides and peptidomimetics vol.E22a, p425-588, Houben-Weyl et al., Goodman Editor, Thieme-Stuttgart-New York (2004), et, parmi ces exemples, peuvent être notamment citées les méthodes d'activation des acides carboxyliques via un azoture (azoture d'acyle), par exemple par l'action d'un réactif tel que l'azoture de diphénylphosphoryle (communément désigné par l'acronyme anglais DPPA), l'utilisation des carbodiimides seules ou en présence de catalyseurs (par exemple le N-hydroxysuccinimide et ses dérivés), l'utilisation d'un carbonyldiimidazole (1,1'-carbonyldiimidazole, CDI), l'utilisation des sels de phosphonium comme l'hexafluorophosphate de benzotriazol-1-yloxy-tris(dimethylamino)phosphonium (communément désigné par l'acronyme anglais BOP) ou encore les uroniums comme l'hexafluorophosphate de 2-(1H-benzotriazol-1-yl)-1,1,3,3-tétraméthyluronium (communément désigné par l'acronyme anglais HBTU).

De préférence, l'étape c) comprend l'activation des fonctions acides carboxyliques (-COOH) susmentionnées sous la forme de fonctions esters, chlorures d'acyle ou anhydrides d'acide.

Ce mode de réalisation est préféré au couplage peptidique par activation de la fonction acide carboxylique à l'aide d'un agent de couplage tel que EDCI/HOBT comme décrit dans le document EP 1 931 673. En effet, un tel couplage entraîne la formation d'un équivalent de 1-éthyl-3-[3-(diméthylamino)propyl]urée, qui doit être éliminé, notamment par chromatographie sur silice ou par extraction liquide/liquide en ajoutant un solvant. Indépendamment de la complexification du procédé engendrée par une telle étape additionnelle, la mise en oeuvre de telles méthodes de purification n'est pas souhaitable, comme discuté précédemment. De plus, l'usage de HOBT est en soit problématique, en cela qu'il s'agit d'un produit explosif.

Par « fonction ester », on entend désigner un groupement -C(O)O-. Il peut s'agir en particulier d'un groupement -C(O)O-R₁, dans lequel R₁ correspond à un groupement (C₁-C₆)alkyle.

Par groupement « (C₁-C₆)alkyle », on entend une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « fonction chlorure d'acyle », également appelée « fonction chlorure d'acide », on entend désigner un groupement -CO-CI.

Par « fonction anhydride d'acide », on entend désigner un groupement -CO-O-CO-. Il peut s'agir en particulier d'un groupement -CO-O-CO-R₂, dans lequel R₂ correspond à un groupement (C₁-C₆)alkyle.

Les réactions de conversion d'une fonction acide carboxylique en fonction ester, chlorure d'acyle ou anhydride d'acide sont bien connues de l'homme du métier, qui pourra les mettre en oeuvre selon toute méthode usuelle dont il est familier.

Le complexe de formule (II) est ensuite obtenu par aminolyse des fonctions acides carboxyliques activées sous la forme de fonctions esters, chlorures d'acyle ou anhydrides d'acide, notamment esters ou anhydrides d'acide, de préférence esters, par réaction avec le 3-amino-1,2-propanediol, sous forme racémique ou énantiomériquement pure, de préférence sous forme racémique.

De manière préférentielle, les étapes d'activation des fonctions acides carboxyliques et d'aminolyse sont effectuées selon un mode de réalisation monotope (ou « one-pot » en anglais), c'est-à-dire dans le même réacteur et sans étape intermédiaire d'isolement ou de purification de l'intermédiaire comportant les fonctions acides carboxyliques activées sous la forme de fonctions esters, chlorures d'acyle ou anhydrides d'acide, notamment esters ou anhydrides d'acide, de préférence esters.

Selon un mode de réalisation particulier du procédé non revendiqué, l'étape c) comprend les étapes successives suivantes :
**c1)** formation d'un complexe activé de formule (VII), 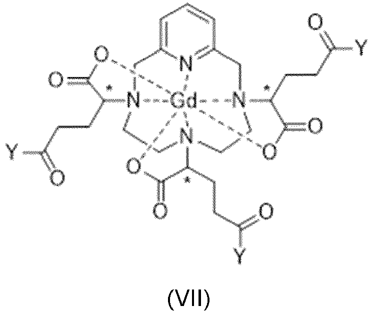 dans laquelle Y représente un atome de chlore, un groupement -OR₁ ou -O-C(O)-R₂, de préférence Y représente un groupement -OR₁ ou -O-C(O)-R₂, avec R₁ et R₂ correspondant, indépendamment l'un de l'autre, à un groupe (C₁-C₆)alkyle, et
**c2)** aminolyse du complexe activé de formule (VII) avec le 3-amino-1,2-propanediol.

Comme cela apparaîtra clairement à l'homme du métier, la réaction de formation du complexe activé de formule (VII) ne modifie pas la configuration absolue des trois atomes de carbones asymétriques situés en position α sur les chaînes latérales, par rapport aux atomes d'azote du macrocycle sur lesquels sont greffées lesdites chaînes latérales. Par conséquent, l'étape c1) permet d'obtenir le complexe activé de formule (VII) avec un excès diastéréoisomérique comprenant un mélange des isomères VII-RRR et VII-SSS, de formules (VII-RRR) et (VII-SSS) représentées ci-après, identique à l'excès diastéréoisomérique comprenant un mélange des isomères I-RRR et I-SSS avec lequel est obtenu le complexe de gadolinium d'hexaacide de formule (I) diastéréoisomériquement enrichi obtenu à l'issue de l'étape b), qui est d'au moins 80 %.

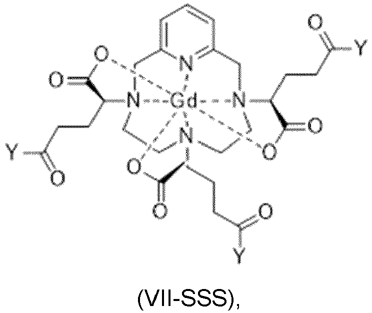

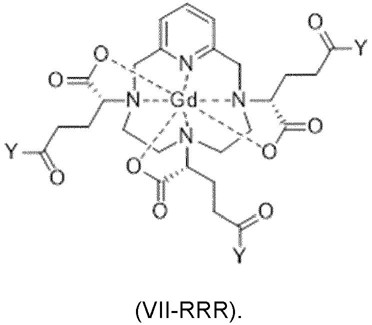

Dans le cas où Y représente un atome de chlore, l'étape c1) est typiquement réalisée par réaction entre le complexe de gadolinium d'hexaacide de formule (I) diastéréoisomériquement enrichi obtenu lors de l'étape b) et le chlorure de thionyle (SOCl₂).

Dans le cas où Y représente un groupement -O-C(O)-CH₃, l'étape c1) est typiquement réalisée par réaction entre le complexe de gadolinium d'hexaacide de formule (I) diastéréoisomériquement enrichi obtenu lors de l'étape b) et le chlorure d'acétyle.

Dans un mode de réalisation avantageux du procédé non revendiqué, l'étape c) comprend l'activation des fonctions acides carboxyliques (-COOH) susmentionnées sous la forme de fonctions esters.

Selon ce mode de réalisation, l'étape c) peut plus particulièrement comprendre les étapes successives suivantes :
**c1)** formation d'un triester de formule (VIII), 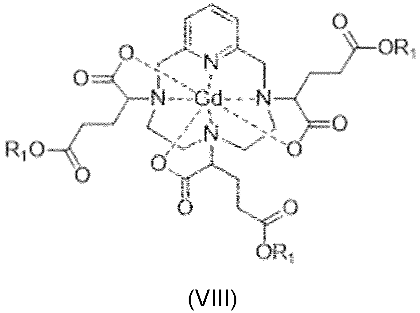 dans laquelle R₁ représente un groupe (C₁-C₆)alkyle, et
**c2)** aminolyse du triester de formule (VIII) avec le 3-amino-1,2-propanediol.

L'étape c1) est typiquement réalisée dans l'alcool de formule R,OH, qui joue à la fois le rôle de solvant et de réactif, en présence d'un acide tel que l'acide chlorhydrique.

L'étape c2) est également typiquement réalisée dans l'alcool de formule R,OH, en présence d'un acide tel que l'acide chlorhydrique.

Dans un premier temps, le complexe de gadolinium d'hexaacide de formule (I) et l'alcool RiOH sont chargés dans le réacteur. Le milieu réactionnel est ensuite refroidi à une température inférieure à 10°C, notamment inférieure à 5 °C, typiquement à 0°C, et une solution acide de l'alcool R,OH, typiquement d'acide chlorhydrique dans RiOH est alors progressivement ajoutée. Le milieu réactionnel est maintenu sous agitation à température ambiante (c'est-à-dire à une température entre 20 et 25°C) pendant une durée typiquement supérieure à 5h, de préférence comprise entre 10h et 20h. Le milieu réactionnel est refroidi à une température inférieure à 10°C, notamment comprise entre 0°C et 5 °C, préalablement à l'étape c2).

Ainsi, les étapes c1) et c2) peuvent être aisément mises en oeuvre selon un mode de réalisation monotope (ou « one-pot » en anglais). Avantageusement, le triester de formule (VII) n'est pas isolé entre les étapes c1) et c2).

Toutefois, afin de favoriser la réaction d'aminolyse, au cours de l'étape c2), l'alcool de formule RiOH est de préférence éliminé par distillation sous vide.

Par « distillation sous vide », on entend, au sens de la présente description, la distillation d'un mélange réalisée à une pression comprise entre 10 et 500 mbar, notamment entre 10 et 350 mbar, de préférence entre 10 et 150 mbar, en particulier entre 50 et 100 mbar.

De même, afin de favoriser la réaction d'aminolyse, lors de l'étape c2), le 3-amino-1,2-propanediol est introduit en large excès. Typiquement, la quantité de matière de 3-amino-1,2-propanediol introduite est supérieure à 4 éq., notamment supérieure 7 éq., avantageusement supérieure à 10 éq., par rapport à la quantité de matière de complexe de gadolinium d'hexaacide de formule (I) diastéréoisomériquement enrichi initialement introduite au cours de l'étape c), qui correspond quant à elle à 1 équivalent.

De manière surprenante, en dépit des conditions acides typiquement employées au cours des étapes c1) et c2), qui devraient augmenter l'instabilité cinétique des complexes de gadolinium, on n'observe pas de décomplexation ou d'isomérisation du triester de formule (VIII). Le triamide désiré est obtenu avec un très bon taux de conversion et la configuration absolue des trois atomes de carbones asymétriques situés en position α sur les chaînes latérales, par rapport aux atomes d'azote du macrocycle, est conservée.

Par ailleurs, il est à noter que, de manière générale, les réactions d'amidification par réaction directe entre un ester et une amine sont très peu décrites dans la littérature (voir à ce sujet K. C. Nadimpally et al., Tetrahedron Letters, 2011, 52, 2579-2582).

Dans un mode de réalisation préféré du procédé non revendiqué, l'étape c) comprend les étapes successives suivantes :
**c1)** formation d'un triester de méthyle de formule (IV), 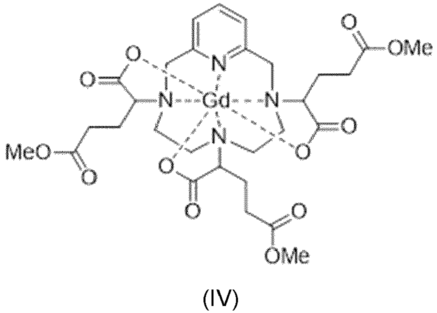 notamment par réaction dans du méthanol en présence d'un acide tel que l'acide chlorhydrique, et
c2) aminolyse du triester de méthyle de formule (IV) avec le 3-amino-1,2-propanediol, notamment dans du méthanol en présence d'un acide tel que l'acide chlorhydrique.

Avantageusement, le triester de méthyle de formule (IV) n'est pas isolé entre les étapes c1) et c2).

Dans un mode de réalisation préféré du procédé non revendiqué, au cours de l'étape c2), le méthanol est éliminé par distillation sous vide, jusqu'à atteindre une température typiquement supérieure à 55°C, notamment comprise entre 60°C et 65°C, et le milieu réactionnel est maintenu à cette température sous vide pendant une durée typiquement supérieure à 5h, notamment comprise entre 10h et 20h, avant d'être refroidi à température ambiante et dilué avec de l'eau.

### ▪ Préparation de l'hexaacide de formule (III)

L'hexaacide de formule (III), qui intervient lors de l'étape a) du procédé de préparation permettant d'obtenir le complexe de formule (II) d'une composition selon l'invention, peut être préparé selon toutes les méthodes d'ores et déjà connues et notamment selon les méthodes décrites dans le brevet EP 1 931 673.

Toutefois, selon un mode de réalisation préféré du procédé non revendiqué, l'hexaacide de formule (III) est obtenu par alkylation du pyclène de formule (V) : 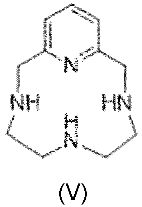 avec un composé de formule R₃OOC-CHGₚ-(CH₂)₂-COOR₄ (IX),
dans laquelle :
- R₃ et R₄ représentent, indépendamment l'un de l'autre, un groupe (C₃-C₆)alkyle, notamment un groupe (C₄-C₆)alkyle tel qu'un groupement butyle, isobutyle, sec-butyle, *tert*-butyle, pentyle ou encore hexyle, et
- Gₚ représente un groupe partant tel qu'un groupement tosylate, triflate, ou un atome d'halogène, de préférence un atome de brome,
   pour obtenir l'hexaester de formule (X) 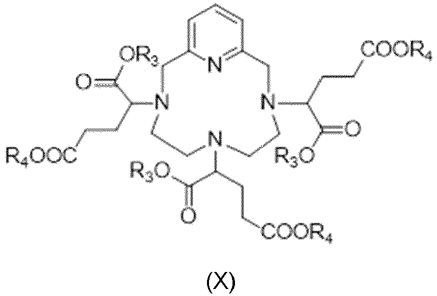 suivie d'une étape d'hydrolyse, conduisant audit hexaacide de formule (III).

Dans un mode de réalisation préféré du procédé non revendiqué, R₃ et R₄ sont identiques.

Selon un mode de réalisation avantageux du procédé non revendiqué, l'hexaacide de formule (III) est obtenu par alkylation du pyclène de formule (V) : 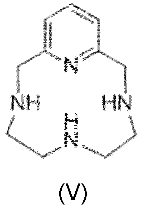 avec le 2-bromoglutarate de dibutyle, pour obtenir l'hexaester de butyle de formule (VI) : 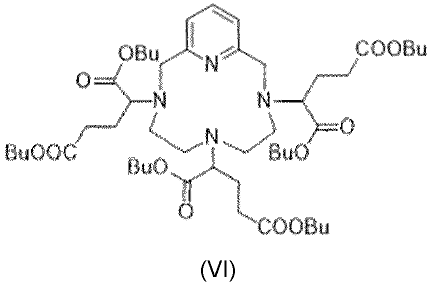 suivie d'une étape d'hydrolyse, conduisant audit hexaacide de formule (III).

Le 2-bromoglutarate de dibutyle utilisé est sous forme racémique ou énantiomériquement pure, de préférence sous forme racémique.

L'utilisation du 2-bromoglutarate de dibutyle est particulièrement avantageuse, en comparaison à celle du 2-bromoglutarate d'éthyle décrite dans le document EP 1 931 673. En effet, le 2-bromoglutarate de diéthyle commercial est un composé relativement instable, qui se dégrade dans le temps et sous l'effet de la température. Plus précisément, cet ester a tendance à s'hydrolyser ou cycliser et donc à perdre son atome de brome. Les tentatives de purification du 2-bromoglutarate de diéthyle commercial, ou de mise au point de nouvelles voies de synthèse permettant de l'obtenir avec une pureté améliorée, et ainsi empêcher sa dégradation, n'ont pas abouti.

La réaction d'alkylation est typiquement réalisée dans un solvant polaire, de préférence dans l'eau, en particulier dans de l'eau désionisée, avantageusement en présence d'une base telle que le carbonate de potassium ou de sodium.

L'utilisation d'eau est préférée notamment à celle d'acétonitrile, décrite dans le document EP 1 931 673, pour des raisons évidentes.

La réaction est avantageusement conduite à une température comprise entre 40°C et 80°C, typiquement entre 50°C et 70°C, notamment entre 55°C et 60°C, pendant une durée comprise entre 5h et 20h, en particulier entre 8h et 15h.

L'étape d'hydrolyse est réalisée avantageusement en présence d'un acide ou d'une base, avantageusement d'une base telle que la soude. Le solvant d'hydrolyse peut être de l'eau, un alcool tel que l'éthanol, ou un mélange eau/alcool. Cette étape est conduite avantageusement à une température comprise entre 40°C et 80°C, typiquement entre 40°C et 70°C, notamment entre 50°C et 60°C, typiquement pendant une durée comprise entre 10h et 30h, en particulier entre 15h et 25h.

### Procédé de purification du complexe de formule (II)

Le complexe de formule (II) d'une composition selon l'invention peut notamment être purifié par un procédé comprenant :
1) la combinaison des 2 étapes suivantes :
   1b) passage sur résine(s) échangeuse(s) d'ions, et
   1c) ultrafiltration dudit complexe, et
2) l'isolement du complexe purifié ainsi obtenu sous forme solide.

Ce procédé de purification n'est pas couvert par le texte des revendications. Sa description détaillée ci-après est néanmoins considérée comme utile à la compréhension de l'invention.

Avantageusement, ledit complexe de formule (II) ayant au moins 80 %, préférentiellement au moins 85 %, notamment au moins 90 %, en particulier au moins 95 %, plus particulièrement au moins 97 %, de préférence au moins 98 %, avantageusement au moins 99 %, d'un excès diastéréoisomérique comprenant un mélange d'isomères II-RRR et II-SSS a été préalablement obtenu selon le procédé de préparation précédemment décrit.

Dans un mode de réalisation préféré du procédé non revendiqué, le complexe diastéréoisomériquement enrichi sur lequel est mis en oeuvre le procédé de purification présente au moins 85 %, notamment au moins 90 %, en particulier au moins 92 %, de préférence au moins 94 %, avantageusement au moins 97 %, plus avantageusement au moins 99 % de l'excès diastéréoisomérique comprenant le mélange d'isomères II-RRR et II-SSS.

De préférence, ledit excès diastéréoisomérique est constitué d'au moins 90 %, de préférence d'au moins 95 % du mélange d'isomères II-RRR et II-SSS.

Avantageusement, ledit excès diastéréoisomérique consiste en le mélange d'isomères II-RRR et II-SSS.

Le terme « mélange d'isomères II-RRR et II-SSS » recouvre également, par extension, le cas où seul l'un des isomères, qu'il s'agisse du II-RRR ou du II-SSS, est présent. Toutefois, le terme « mélange d'isomères II-RRR et II-SSS » désigne préférentiellement l'ensemble des cas où chacun des isomères II-RRR et II-SSS est présent en une quantité variable mais non nulle.

Dans un mode de réalisation préféré, les isomères II-RRR et II-SSS sont présents au sein dudit mélange dans un rapport compris entre 65/35 et 35/65, notamment entre 60/40 et 40/60, en particulier entre 55/45 et 45/55. Avantageusement, les isomères II-RRR et II-SSS sont présents au sein du mélange dans un rapport 50/50.

### ▪ Combinaison des étapes 1b) et 1c)

Les étapes 1b) et 1c) visent à purifier le complexe de formule (II) en éliminant les impuretés éventuellement présentes du fait de son procédé d'obtention.

Lesdites impuretés peuvent notamment comprendre le 3-amino-1,2-propanediol et/ou une impureté dicouplée.

En effet, du 3-amino-1,2-propanediol peut être présent dans le produit final obtenu lors de la mise en oeuvre d'un procédé de préparation du complexe de formule (II), typiquement lorsque le complexe de formule (II) est obtenu par amidification à partir du complexe de formule (I) et du 3-amino-1,2-propanediol. Comme détaillé précédemment, la réaction d'amidification peut comprendre l'activation des trois fonctions acides carboxyliques portées par les atomes de carbone situés en position γ sur les chaînes latérales du complexe de formule (I), par rapport aux atomes d'azote du macrocycle sur lesquels sont greffées lesdites chaînes latérales, suivie d'une aminolyse des fonctions acides carboxyliques activées par réaction avec le 3-amino-1,2-propanediol. Le 3-amino-1,2-propanediol est alors avantageusement employé en excès, de façon à assurer une bonne conversion en fonctions amides des trois fonctions acides carboxyliques activées.

Par « impureté dicouplée », on entend désigner un complexe de formule (II-dc-a), (II-dc-b), (II-dc-c) représentées ci-dessous ou un mélange de ceux-ci :

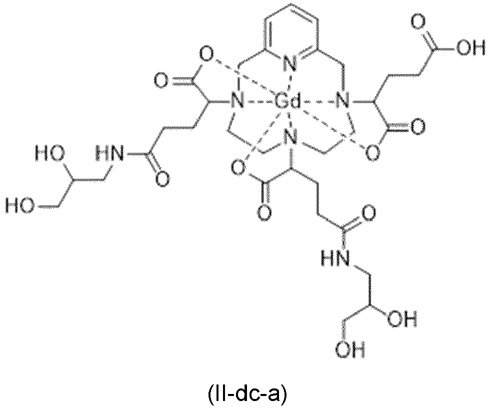

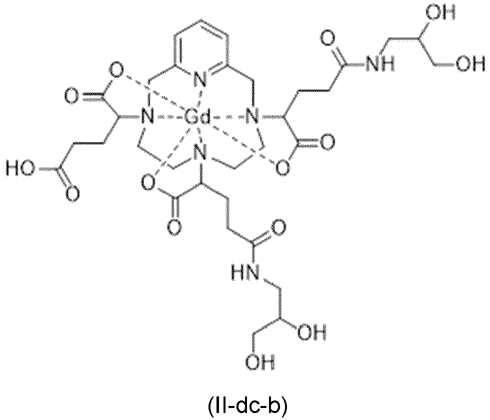

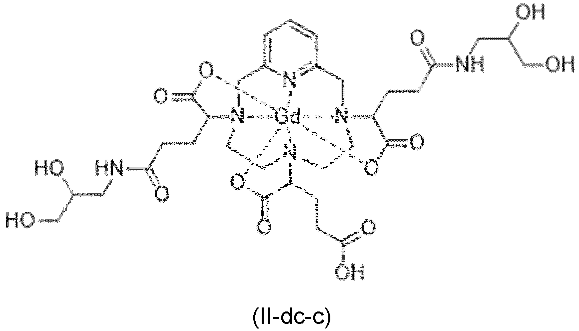

L'impureté dicouplée peut notamment résulter de la réaction d'hydrolyse d'une fonction amide du complexe de formule (II). Elle peut aussi résulter d'une activation incomplète des fonctions acides carboxyliques du complexe de formule (I) (activation de deux fonctions sur les trois) ou d'une aminolyse incomplète des fonctions acides carboxyliques activées (aminolyse de deux fonctions sur les trois), lorsque le procédé de préparation du complexe de formule (II) met en oeuvre de telles étapes.

### ▪ L'étape 1b) correspond au passage sur résine(s) échangeuse(s) d'ions du complexe de formule (II) diastéréoisomériquement enrichi tel que décrit précédemment.

Par « résine échangeuse d'ions », on entend, au sens de la présente description, un matériau solide se présentant généralement sous forme de billes composées d'une matrice polymère sur laquelle sont greffés des groupements fonctionnels chargés positivement (résine anionique) ou négativement (résine cationique), qui vont permettre de piéger respectivement des anions ou cations par adsorption. L'adsorption des anions ou cations sur la résine procède par échange d'ions entre les contre-ions des groupements fonctionnels initialement présents afin d'assurer l'électroneutralité de la résine, et les anions ou cations destinés à être piégés.

L'étape 1b) comprend la mise en contact d'une solution aqueuse du complexe de formule (II) diastéréoisomériquement enrichi avec une résine anionique forte. L'eau utilisée est de préférence une eau purifiée.

Ladite résine anionique forte comporte typiquement en tant que groupements fonctionnels échangeurs des groupes ammoniums (N(RR'R")⁺, où R, R' et R" sont des groupements (C₁-C₆)alkyle identiques ou différents). On peut notamment citer la résine Amberlite^{®} FPA900 commercialisée par Dow Chemical, avantageusement sous forme HO⁻.

Le passage sur résine anionique forte permet d'éliminer, au moins en partie, les impuretés dicouplées.

L'étape 1b) peut comprendre en outre la mise en contact d'une solution aqueuse du complexe de formule (II) diastéréoisomériquement enrichi avec une résine cationique faible. L'eau utilisée est de préférence une eau purifiée.

Ladite résine cationique faible comporte typiquement en tant que groupements fonctionnels échangeurs des groupes carboxylates (CO₂⁻). On peut notamment citer la résine IMAC^{®} HP336 commercialisée par Dow Chemical, avantageusement sous forme H⁺.

Le passage sur résine cationique faible permet d'éliminer, au moins en partie, le 3-amino-1,2-propanediol, et les éventuels résidus de Gd³⁺.

Il est à noter que l'étape 1b) de passage sur résine(s) échangeuse(s) d'ions est rendue possible par la stabilité améliorée du complexe de formule (II) diastéréoisomériquement enrichi, dont l'intégrité est dès lors préservée lors de cette étape.

### ▪ L'étape 1c) correspond à l'ultrafiltration du complexe de formule (II) diastéréoisomériquement enrichi tel que décrit précédemment.

Par « ultrafiltration », on entend désigner, dans la présente description, une méthode de filtration au travers d'une membrane semi-perméable, mésoporeuse, dont les pores ont généralement un diamètre compris entre 1 et 100 nm, en particulier entre 2 et 50 nm, notamment entre 10 et 50 nm (mésopores), sous l'effet de forces telles que des gradients de pression, typiquement entre 1 et 10 bars, et éventuellement de concentration. Il s'agit donc d'un procédé de séparation membranaire par laquelle des particules en solution ou en suspension dont la taille est supérieure à celle des pores sont retenues par la membrane, et séparées du mélange liquide qui les contenait.

Dans le cadre du procédé de purification d'un complexe de formule (II) d'une composition selon l'invention, l'ultrafiltration est particulièrement avantageuse pour éliminer les endotoxines.

Avantageusement, la membrane d'ultrafiltration utilisée lors de l'étape 1c) a un seuil de coupure inférieur à 100 kD, notamment inférieur à 50 kD, en particulier inférieur à 25 kD, typiquement, un seuil de coupure de 10 kD.

De préférence, lors de l'étape 1c), la pression transmembranaire est comprise entre 1 et 5 bars, en particulier entre 2,25 et 3,25 bars.

### ▪ Dans un mode de réalisation particulier du procédé non revendiqué, les étapes 1b) et 1c) sont en outre combinées à une étape 1a) de nanofiltration.

Par « nanofiltration », on entend désigner, dans la présente description, une méthode de filtration au travers d'une membrane semi-perméable, poreuse, dont les pores ont généralement un diamètre compris entre 0,1 et 100 nm, en particulier entre 0,1 et 20 nm notamment entre 1 et 10 nm, sous l'effet de forces telles que des gradients de pression, typiquement entre 1 et 50 bars, et éventuellement de concentration. Il s'agit donc d'un procédé de séparation membranaire par laquelle des particules en solution ou en suspension dont la taille est supérieure à celle des pores sont retenues par la membrane, et séparées du mélange liquide qui les contenait.

L'étape 1a) de nanofiltration permet d'éliminer l'essentiel du 3-amino-1,2-propanediol (éventuellement sous forme de sel, en particulier de chlorhydrate, ou de dérivés, notamment le dérivé acétamide) en excès et les sels minéraux.

Dans ce mode de réalisation particulier du procédé non revendiqué, l'étape de nanofiltration peut être réalisée directement sur le complexe de formule (II) diastéréoisomériquement enrichi brut tel qu'obtenu selon le procédé de préparation précédemment décrit. Il n'est notamment pas nécessaire de précipiter le complexe de formule (II) diastéréoisomériquement enrichi préalablement préparé par ajout de solvant.

Avantageusement, la membrane de nanofiltration utilisée lors de l'étape 1a) a un seuil de coupure inférieur à 1kD, notamment inférieur à 500 Daltons, en particulier inférieur à 300 Daltons, typiquement, un seuil de coupure de 200 Daltons.

De préférence, lors de l'étape 1a), la pression transmembranaire est comprise entre 10 et 40 bars, en particulier entre 2 et 30 bars.

En particulier, la température de la solution du complexe de formule (II) soumise à ultrafiltration lors de l'étape 1a) est comprise entre 20 et 40°C, notamment entre 25 et 35°C.

Dans une alternative de ce mode de réalisation particulier, l'étape 1b) ne comprend pas la mise en contact d'une solution aqueuse du complexe de formule (II) diastéréoisomériquement enrichi avec une résine cationique faible.

Dans un mode de réalisation particulier, les étapes 1a) lorsque celle-ci est présente, 1b et 1c sont effectuées dans cet ordre. Ce mode de réalisation avantageux permet notamment de minimiser les quantités de résines utilisées et donc le coût de fabrication industrielle.

### ▪ Etape 2)

L'étape 2) vise à isoler sous forme solide le complexe de formule (II) purifié obtenu à l'issue de la combinaison des étapes 1b) et 1c), et optionnellement combinées en outre à l'étape 1a).

Cette étape d'isolement sous forme solide peut être réalisée selon toute méthode bien connue de l'homme du métier, notamment par atomisation, par précipitation, par lyophilisation ou par centrifugation, avantageusement par atomisation.

Dans un mode de réalisation préféré du procédé non revendiqué, l'étape 2) comprend une atomisation.

En effet, l'isolement sous forme solide du complexe de formule (II) purifié par atomisation permet notamment de s'affranchir de l'utilisation de solvants de précipitation.

La température d'entrée de l'air dans l'atomiseur est alors typiquement comprise entre 150°C et 180°C, notamment entre 160°C et 175°C, avantageusement entre 165°C et 170°C. La température de sortie est quant à elle typiquement comprise entre 90°C et 120°C, de préférence entre 105°C et 110°C.

Avantageusement, le degré de pureté du complexe de formule (II) diastéréoisomériquement enrichi en le mélange d'isomères I I-RRR et II-SSS purifié et isolé à l'issue de l'étape 2) est supérieur à 95 %, notamment supérieur à 97 %, préférentiellement supérieur à 97,5 %, plus préférentiellement supérieur à 98 %, avantageusement supérieur à 99 %, ledit degré de pureté étant exprimé en pourcentage massique du complexe de formule (II) par rapport à la masse totale obtenue à l'issue de l'étape 2).

De manière préférée, le complexe de formule (II) compris dans la composition selon l'invention précédemment décrite est le complexe de formule (II) diastéréoisomériquement enrichi et purifié, susceptible d'être obtenu selon le procédé de purification de l'invention.

### EXEMPLES

Les exemples figurant ci-après sont présentés à titre illustratif et non limitatif de l'invention.

### Séparation des groupes d'isomères iso1, iso2, iso3 et iso4 du complexe de formule (II) par UHPLC

Un appareil UHPLC constitué d'un système de pompage, d'un injecteur, d'une colonne chromatographique, d'un détecteur UV et d'une station de données est utilisé. La colonne chromatographique utilisée est une colonne UHPLC 150 x 2,1 mm -1,6 µm (colonne CORTECS^{®} UPLC T3 de Waters).
- Phase mobile :
Voie A : 100% acétonitrile et Voie B : solution aqueuse d'H₂SO₄ (96%) à 0,0005 % v/v
- Préparation des solutions essais :
Solution du complexe de formule (II) à 2 mg/mL dans de l'eau purifiée
- Conditions d'analyse :

| | |
|---|---|
| Température colonne | 40°C |
| Température échantillon | Température ambiante (20-25°C) |
| Débit | 0,3 ml/min |
| Volume d'injection | 1 µl |
| Détection UV | 200 nm |
| Durée d'analyse | 20 min |

- Gradient :

| Temps | % Acn | % H₂SO₄ 0,0005 % |
|---|---|---|
| 0 | 1 | 99 |
| 3 | 5 | 95 |
| 12 | 10 | 90 |
| 15 | 25 | 75 |
| 16 | 1 | 99 |
| 20 | 1 | 99 |

4 pics principaux sont obtenus. Le pic 4 de la trace UHPLC, à savoir l'iso4, correspond à un temps de rétention de 6,3 minutes.

### Préparation de l'hexaester de butyle de formule (VI)

Dans un réacteur, 184 kg (570 moles) de 2-bromoglutarate de dibutyle et 89 kg (644 moles) de carbonate de potassium sont mélangés et chauffés à 55-60°C. Une solution aqueuse de 29,4 kg (143 moles) de pyclène dans 24 kg d'eau est ajoutée à la préparation précédente. Le mélange réactionnel est maintenu à 55-60°C puis est chauffé à reflux une dizaine d'heures. Après réaction, le milieu est refroidi, dilué avec 155 kg de toluène puis lavé avec 300 litres d'eau. L'hexaester de butyle est extrait en phase aqueuse avec 175 kg (1340 moles) d'acide phosphorique (75%). Il est ensuite lavé 3 fois avec 150 kg de toluène. L'hexaester de butyle est reextrait en phase toluènique par dilution avec 145 kg de toluène et 165 kg d'eau suivie d'une basification à la soude 30% (m/m) pour atteindre un pH de 5-5,5. La phase aqueuse inférieure est éliminée. L'hexaester de butyle est obtenu par concentration à sec sous vide à 60°C avec un rendement d'environ 85%.

### Préparation de l'hexaacide de formule (III)

Dans un réacteur, 113 kg (121 moles) d'hexaester de butyle sont chargés ainsi que 8 kg d'éthanol. Le milieu est porté à 55+/-5°C puis 161 kg (1207.5 moles) de soude 30 % (m/m) sont coulés en 3 heures. Le mélange réactionnel est maintenu à cette température pendant une vingtaine d'heures. Le butanol est ensuite retiré par décantation du milieu réactionnel. L'hexaacide de formule (III) obtenu sous forme de sel de sodium est dilué avec de l'eau pour obtenir une solution aqueuse d'environ 10 % (m/m). Cette solution est traitée sur une résine cationique acide. L'hexaacide de formule (III) en solution aqueuse est obtenu avec un rendement d'environ 90 % et une pureté de 95 %.

### Préparation du complexe de gadolinium d'hexaacide de formule (I)

### ▪ Protocole Expérimental

### • Complexation et isomérisation

### - Sans acide acétique

Dans un réacteur 418 kg (117 kg d'hexaacide de formule (III) pur / 196 moles) d'une solution aqueuse d'hexaacide de formule (III) à 28 % en poids sont chargés. Le pH de la solution est ajusté à 2,7 par ajout d'acide chlorhydrique, puis 37 kg (103,2 moles) d'oxyde de gadolinium sont ajoutés. Le milieu réactionnel est chauffé à 100-102°C pendant 48H pour atteindre la répartition isomérique attendue de l'hexaacide de formule (III).

### - Avec acide acétique

De l'oxyde de gadolinium (0,525 éq. molaire) est mis en suspension dans une solution d'hexaacide de formule (III) à 28,1 % massique.

L'acide acétique 99-100 % (50 % massique / hexaacide de formule (III) pur) est coulé sur le milieu à température ambiante.

Le milieu est chauffé à reflux puis distillation jusqu'à 113°C en masse en rechargeant le milieu avec de l'acide acétique au fur et à mesure de l'élimination de l'eau. Arrivé à 113°C, on rajoute la quantité suffisante d'acide acétique afin d'arriver au volume de départ.

Le milieu est maintenu à 113°C pendant une nuit.

### • Cristallisation, Recristallisation

### - Cristallisation

Le complexe de gadolinium d'hexaacide de formule (I) en solution est refroidi à 40°C, l'amorce est ajoutée, on laisse en contact pendant au moins 2h. Il est ensuite isolé par filtration à 40°C et lavé avec de l'eau osmosée.

### - Recristallisation

180 kg du complexe de gadolinium d'hexaacide de formule (I) obtenu précédemment (extrait sec à environ 72 %) sont mis en suspension dans 390 kg d'eau. Le milieu est chauffé à 100°C pour solubiliser le produit, puis refroidi à 80°C pour être amorcé en ajoutant un peu d'amorce. Après refroidissement à température ambiante le complexe de gadolinium d'hexaacide de formule (I) est isolé par filtration et séchage.

### • Décomplexation sélective

Le produit sec est chargé dans le réacteur avec de l'eau osmosée/ à 20°C. La masse d'eau ajoutée est égale au double de la masse de complexe de gadolinium d'hexaacide de formule (I) théorique. De la soude 30,5 % (m/m) (6,5 éq) est coulée sur le milieu à 20°C. On laisse le milieu en contact à 50°C à la fin de l'ajout de NaOH pendant 16h00. Le milieu est refroidi à 25°C et le produit filtré sur un lit de Clarcel.

### ▪ Teneur en le mélange de diastéréoisomères I-RRR et I-SSS

Le rapport dans lequel les différents isomères du complexe de formule (I) sont présents au sein du mélange de diastéréoisomères dépend des conditions dans lesquelles sont effectuées les étapes de complexation et d'isomérisation, comme cela apparaît dans le tableau 3 ci-dessous.

**Tableau 3 : teneur en le mélange I-RRR et I-SSS en fonction des confitions de complexation / isomérisation**

| *pH* | *Température* | *Teneur en hexaacide de formule (III)* | *Durée* | *excès diastéréoisomérique en le mélange I-RRR et I*-*SSS* |
|---|---|---|---|---|
| 5,7 | 80°C | 40 % | 3h | 19 % |
| 3,5 | 90°C | 50 % | 10h | 49 % |
| 3,0 | 101°C | 40 % | 10h | 68 % |
| 2,7 | 101°C | 28 % | 48h | 98,04 % |

Les étapes additionnelles de recristallisation et décomplexation sélective permettent d'augmenter l'excès diastéréoisomérique en le mélange I-RRR et I-SSS (voir tableau 4).

**Tableau 4 : teneur en le mélange I-RRR et I-SSS après cristallisation / recristallisation / décomplexation sélective**

| | *Après la 1^{ère} cristallisation* | *Après recristallisation* | *Après décomplexation sélective* |
|---|---|---|---|
| *excès diastéréoisomérique en le mélange I-RRR et I-SSS* | 98,04 % | 99,12 % | 99,75 % |

### Préparation du complexe de formule (II)

Dans un réacteur, 90 kg (119 moles) du complexe d'hexaacide de formule (I) et 650 kg de méthanol sont chargés. Le mélange est refroidi à environ 0°C puis 111 kg (252 moles) d'une solution d'acide chlorhydrique méthanolique (8,25 % d'HCl dans le méthanol) est coulée en maintenant la température à 0°C. Le milieu réactionnel est porté à température ambiante puis est maintenu sous agitation pendant 16 heures. Après refroidissement à 0-5°C, 120 kg (1319 moles) d'3-amino-1,2-propanediol sont ajoutés. Le milieu réactionnel est ensuite chauffé en distillant le méthanol sous vide jusqu'à atteindre une température de 60-65°C. Le concentrât est maintenu pendant 16 heures à cette température sous vide. En fin de contact, le milieu est dilué avec 607 kg d'eau en refroidissant à température ambiante. La solution du complexe de formule (II) brut est neutralisée avec de l'acide chlorhydrique 20 % (m/m). 978,6 kg de solution sont ainsi obtenus, avec une concentration de 10,3 %, représentant 101 kg de matière. Le rendement obtenu est de 86,5 %, la pureté du complexe de formule (II) est de 92,3 % (HPLC s/s). La quantité d'impuretés dicouplées est de 6,4 % (HPLC s/s).

### Purification du complexe de formule (II)

### • Nanofiltration

La membrane de nanofiltration utilisée a un seuil de coupure à 200 Daltons (Koch Membran System SR3D). Ce traitement s'effectue de la façon suivante :
La solution de complexe de formule (II) brut est chauffée à 30°C. Le nanofiltre est rempli par ladite solution. La pompe est mise en marche d'abord à faible débit pour purger le système, puis le débit de la pompe du nanofiltre est monté progressivement au débit de recirculation souhaité (1,0 m³/h pour une membrane de 2.5 X 40 pouces). Le système est ensuite mis en recirculation totale à 30°C pendant au moins 2 heures pour établir une couche de polarisation. Le milieu est alors passé en diafiltration à 30°C sous 25 bars en maintenant le volume constant par ajout d'eau pure jusqu'à obtenir une conductivité du rétentat inférieure à 1000 µS. En fin de diafiltration, le milieu est concentré jusqu'à obtenir une concentration d'environ 40 % (m/m).

### • Traitement sur résines

La solution de complexe de formule (II) issue de la nanofiltration est diluée à l'eau purifiée sous agitation pour obtenir une solution à 15% (m/m). Cette solution est éluée en série sur 50 litres de résines anioniques fortes (FPA900) sous forme OH⁻ puis sur 50 litres de résines cationiques faibles (HP336) sous forme H⁺ à un débit d'élution moyen de 2V/V/H (2 volumes de solution par volume de résine par heure). Les résines sont ensuite rincées avec environ 450 litres d'eau purifiée jusqu'à l'atteinte d'un indice de réfraction inférieur à 1,3335.

La solution de complexe de formule (II) est ensuite concentrée en chauffant jusqu'à 50-60°C sous un vide de 20 mbar pour atteindre une concentration de 35% (m/m).

### • Ultrafiltration

La membrane d'ultrafiltration est une membrane UF 10KD Koch Spiral.

L'ultrafiltre est alimenté par la solution précédente de complexe de formule (II) à 35 % chauffée à 40°C. L'ultrafiltration est appliquée à un débit de 3m³/H avec une pression transmembranaire de 2,5-3 bars. Plusieurs rinçages du système par 13 litres d'eau purifiée apyrogène sont réalisés jusqu'à atteindre une dilution finale du complexe de formule (II) de 25 % (m/m).

### • Atomisation

Le complexe de formule (II) est obtenu sous forme de poudre par atomisation de la solution précédente de complexe de formule (II) concentrée à 25 %.

L'atomisation s'effectue de la façon suivante :
On équilibre l'atomiseur à l'eau pure apyrogène en réglant la température d'entrée à 165°C - 170°C et en adaptant le débit d'alimentation de telle sorte que la température de sortie soit comprise entre 105 et 110°C.

Puis, on ajoute la solution de complexe de formule (II) concentrée et on ajuste le débit de manière à conserver les paramètres ci-dessus.

On maintient ces conditions opératoires pendant toute l'atomisation, tout en s'assurant du bon comportement de la poudre dans la chambre d'atomisation et en sortie de l'atomiseur. Il faut notamment s'assurer qu'il n'y ait pas de collage du produit.

En fin d'alimentation de l'atomiseur avec la solution, on rince le contenant de ce complexe de formule (II) et l'atomiseur à l'eau pure apyrogène jusqu'à récupération maximum de la poudre.

On obtient un complexe de formule (II) pur à 99,6 %.

Ce degré de pureté a été déterminé par chromatographie liquide en phase inverse.

### Composition selon l'invention et résultats d'études sur celle-ci

### • Exemple de procédé de fabrication conforme à l'invention

Le procédé de fabrication d'une composition selon l'invention est réalisé en suivant les étapes suivantes :
a) 485,1 g (soit 0,5 M) de complexe de formule (II) est dissous dans de l'eau (qs 1 litre) en chauffant la cuve à une température entre 39 et 48°C et en réalisant une forte agitation de la solution jusqu'à complète dissolution de ce complexe dans l'eau. La solution est ensuite refroidie à environ 30°C.
b) 0,404 g (soit 0,2 % mole/mole par rapport à la proportion de complexe ajoutée à l'étape a)) de DOTA (Simafex, France) est ajouté sous agitation à la solution obtenue à l'étape a) via une solution de DOTA à 10 % m/v.
c) Du trométamol (Tris) est ajouté à la solution obtenue à l'étape b) sous agitation. Le pH est ensuite ajusté à une valeur entre 7,2 et 7,7 par ajout sous agitation d'une solution d'acide chlorhydrique.
d) La concentration ciblée (0,5 mol/L) est obtenue par ajout en deux étapes d'eau ppi jusqu'à obtenir une valeur de densité entre 1,198 et 1,219 g/mL.

La composition liquide est ensuite filtrée sur une membrane polyethersulfone et mise dans son contenant final, qui est enfin soumis à une stérilisation à 121°C pendant 15 minutes.

### • Exemple de composition conforme à l'invention

Grâce au procédé décrit ci-dessus est obtenue la formulation suivante :

| **Ingrédients** | **Proportions dans la composition** |
|---|---|
| Complexe de formule (II) | 485,1 g (0,5 M) |
| DOTA** | 0,404 g (1 mM soit 0,2% mol/mol vs complexe) |
| NaOH ou HCl | Qs pH 7,2 à 7,7 |
| Trométamol | 1,211 g |
| Gadolinium libre* | < 1 ppm m/v |
| Eau ppi (prête pour injection) | Qs 1 L |

| | |
|---|---|
| * Mesure effectuée par méthode colorimétrique au xylénol orange **exprimé sur une base anhydre et pure | |

### • Essais de formulation réalisés

Différentes concentrations de trométamol de 0 à 100 mM ont été testées. Les résultats de ces tests ont montré qu'une teneur de 10 mM (0,12 % w/v) était suffisante pour garantir la stabilité du pH de la formulation tout en limitant la formation d'impuretés de dégradation.

Différentes concentrations de DOTA de 0 à 2,5 mM ont été testées. Les résultats de ces tests ont montré qu'une teneur de 1 mM, qui correspond à 0,04 % m/v ou 0,2 % mol/mol permet d'assurer l'absence de libération de Gd libre durant le process et pendant la vie du produit.

### • Etudes de stabilité en conditions accélérées d'une composition selon l'invention

La formulation de l'exemple précédent est analysée juste après sa fabrication (T₀) et après un stockage à 40°C pendant 6 mois après sa fabrication (T+6 mois).
A T₀ :
   - Pureté évaluée par chromatographie* : 99,6 %
   - Concentration en Gd-DOTA : 0,007 % (m/V)
   - Concentration en Gd : en dessous de 0,0001 % (m/V)
   - pH : 7,5
A T+6 mois :
   - Pureté évaluée par chromatographie* : 97,2 %
   - Concentration en Gd-DOTA : 0,014% (m/V) - 0,25 mM
   - Concentration en Gd : en dessous de 0,0001 % (m/V)
   - pH : 7,5
      * chromatographie liquide en phase inverse

Ces résultats démontrent que cette formulation a une bonne stabilité dans le temps.

### • Etudes de stabilité comparatives

La stabilité des compositions ci-dessous a été évaluée au cours du temps. Le terme « PA non optimisé » désigne le principe actif, à savoir le complexe de formule (II), obtenu selon le procédé décrit dans le document EP 1 931 673. Le terme « PA optimisé » désigne quant à lui le complexe de formule (II) diastéréoisomériquement enrichi et purifié obtenu par le procédé selon l'invention.

| | PA (0,5 M) | [DOTA] % mol/mol | Trométamol mM | pHₐⱼᵤₛₜₑₘₑₙₜ |
|---|---|---|---|---|
| C1 | Non optimisé | 0,3 | - | 5,0 |
| C2 | Optimisé | 0,2 | - | 7,5 |
| C3 | Optimisé | 0,1 | - | 7,5 |
| C4 | Optimisé | 0,2 | 10 | 7,5 |
| C5 | Optimisé | 0,1 | 10 | 7,5 |
| C6 | Optimisé | 0,2 | - | 5,0 |
| C7 | Optimisé | 0,1 | - | 5,0 |

| | Gd libre en ppm m/v (xylénol) | | DOTA-Gd en % mol/mol (LC formiate*) | |
|---|---|---|---|---|
| | T 0 | T 6 mois 40°C | T 0 | T 6 mois 40°C |
| C1 | < LOD | 0,18 | 0,27 | 0,3 |
| C2 | < LOD | < LOD | 0,02 | 0,05 |
| C3 | < LOD | < LOD | 0,02 | 0,05 |
| C4 | < LOD | < LOD | 0,02 | 0,05 |
| C5 | < LOD | < LOD | 0,02 | 0,08 |
| C6 | < LOD | < LOD | 0,03 | 0,03 |
| \| C7 | < LOD | < LOD | 0,02 | 0,07 |

| | | | | |
|---|---|---|---|---|
| * LC formiate : méthode chromatographique mettant en jeu une détection fluorimétrique. La séparation s'effectue sur une colonne chromatographique greffée C18 en phase inverse avec une élution en mode gradient. | | | | |

Les résultats rapportés ci-dessus indiquent que la formulation du PA non optimisé avec le DOTA libre n'est pas possible. En effet, l'excipient de chélation est entièrement consommé par la réaction de transligation entre le complexe de formule (II) et le DOTA, et ne peut dès lors plus assurer son rôle de piégeage du Gd³⁺ relargué.

En revanche, le complexe de formule (II) diastéréoisomériquement enrichi et purifié obtenu par le procédé précédemment décrit peut être formulé avec le DOTA libre. On constate en effet l'absence de Gd libre dans la composition à 6 mois, 40°C, et ce quel que soit le pH de la formulation et que des espèces tamponantes soient présentes ou non. En outre, la consommation en excipient de chélation est très faible, puisqu'elle n'excède pas 0,08 % mol/mol.

## Revendications

1. Composition comprenant le complexe de formule (II) suivante : 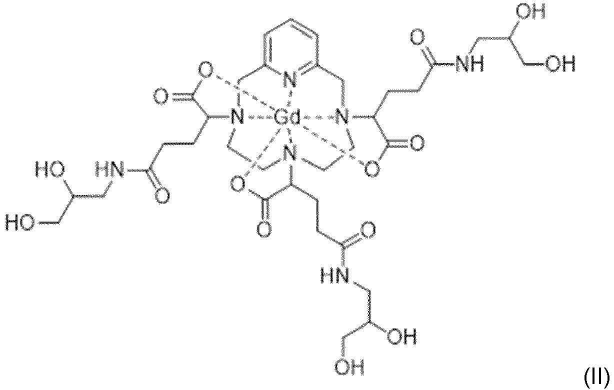 constitué d'au moins 80 % d'un excès diastéréoisomérique comprenant un mélange d'isomères II-RRR et II-SSS de formules : 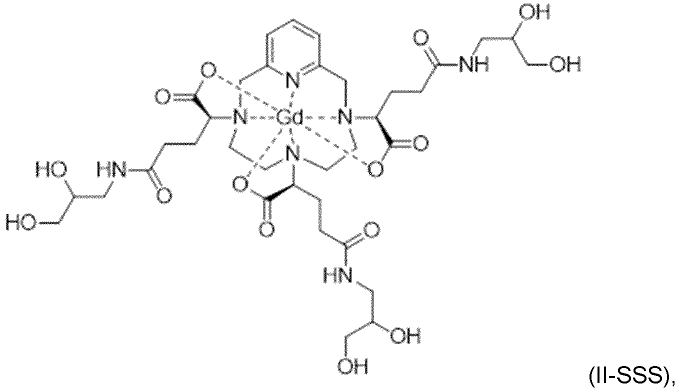 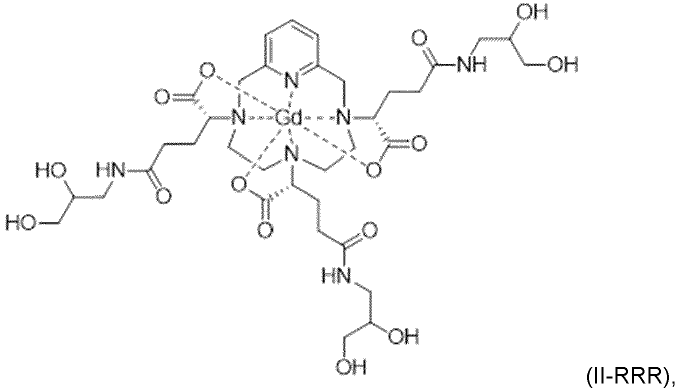 et un ligand macrocyclique libre, et ayant avantageusement une concentration en gadolinium libre inférieure à 1 ppm (m/v), **caractérisée en ce qu'**elle comprend entre 0.002 et 0.4% mol/mol de ligand macrocyclique libre par rapport au complexe de formule (II).

2. Composition selon la revendication 1, **caractérisée en ce que** le complexe de formule (II) présente au moins 90 % de l'excès diastéréoisomérique comprenant le mélange d'isomères II-RRR et II-SSS.

3. Composition selon la revendication 1 ou 2, **caractérisée en ce que** le complexe de formule (II) présente au moins 92 % de l'excès diastéréoisomérique comprenant le mélange d'isomères II-RRR et II-SSS.

4. Composition selon l'une quelconque des revendications 1 à 3, **caractérisée en ce que** le complexe de formule (II) présente au moins 94 % de l'excès diastéréoisomérique comprenant le mélange d'isomères II-RRR et II-SSS.

5. Composition selon l'une quelconque des revendications 1 à 4, **caractérisée en ce qu'**elle comprend entre 0.01 et 0.3% mol/mol de ligand macrocyclique libre par rapport au complexe de formule (II).

6. Composition selon l'une quelconque des revendications 1 à 5, **caractérisée en ce que** le ligand macrocyclique libre est sélectionné dans le groupe constitué de DOTA, NOTA, DO3A, BT-DO3A, HP-DO3A, PCTA, DOTA-GA et leurs dérivés.

7. Composition selon la revendication 6, **caractérisée en ce que** le ligand macrocyclique libre est l'acide 1,4,7,10-tétraazacyclododecane-1,4,7,10-tétraacetique (DOTA).

8. Composition selon l'une quelconque des revendications 1 à 7, **caractérisée en ce qu'**elle présente une concentration comprise entre 0,01 et 1,5 mol.L⁻¹, de préférence entre 0,2 et 0,7 mol.L⁻¹ en complexe de formule (II).

9. Composition selon la revendication 8, **caractérisée en ce que** la concentration en complexe de formule (II) est comprise entre 0,3 et 0,6 mol.L⁻¹.

10. Composition selon l'une quelconque des revendications 1 à 9, **caractérisée en ce que** son pH est compris entre 6,5 et 8.

11. Composition selon l'une quelconque des revendications 1 à 10, **caractérisée en ce qu'**elle comprend le tampon Tris (Tris(hydroxymethyl)aminomethane).

12. Composition selon l'une quelconque des revendications 1 à 11, **caractérisée en ce que** le degré de pureté du complexe de formule (II) est supérieur à 95 %.

13. Composition selon l'une quelconque des revendications 1 à 12, **caractérisée en ce que** le degré de pureté du complexe de formule (II) est supérieur à 97 %.

14. Composition selon la revendication 12 ou 13, **caractérisée en ce que** le ligand macrocyclique libre est le DOTA et **en ce qu'**elle présente une concentration en gadolinium libre inférieure à 1 ppm (m/v).

15. Composition selon la revendication 14, **caractérisée en ce qu'**elle comprend entre 0,002 et 0,4 % mol/mol de DOTA par rapport au complexe de formule (II).

## Patentansprüche

1. Zusammensetzung, umfassend den Komplex der folgenden Formel (II): 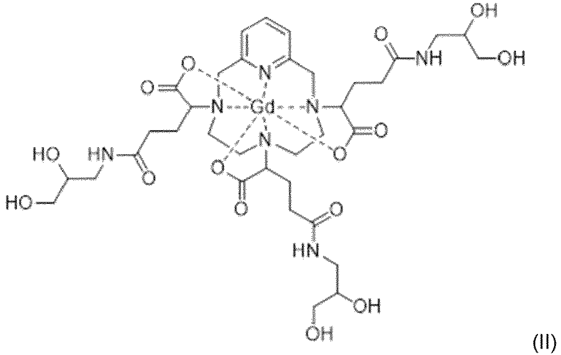 bestehend aus mindestens 80 % eines Diastereoisomerenüberschusses, umfassend ein Gemisch von Isomeren II-RRR und II-SSS der Formeln: 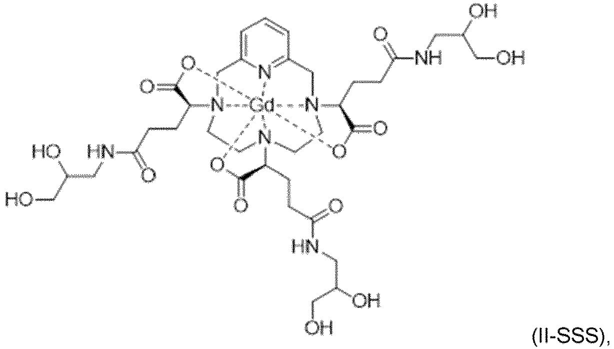 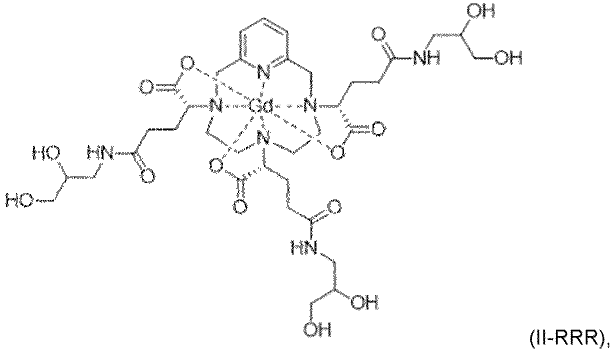 und einen freien makrocyclischen Liganden, und vorteilhafterweise mit einer Konzentration an freiem Gadolinium von weniger als 1 ppm (m/v), **dadurch gekennzeichnet, dass** sie zwischen 0,002 und 0,4 % mol/mol freien makrocyclischen Liganden, bezogen auf den Komplex der Formel (II), umfasst.

2. Zusammensetzung nach Anspruch 1, **dadurch gekennzeichnet, dass** der Komplex der Formel (II) mindestens 90 % des Diastereoisomerenüberschusses, umfassend das Gemisch von Isomeren II-RRR und II-SSS, aufweist.

3. Zusammensetzung nach Anspruch 1 oder 2, **dadurch gekennzeichnet, dass** der Komplex der Formel (II) mindestens 92 % des Diastereoisomerenüberschusses, umfassend das Gemisch von Isomeren II-RRR und II-SSS, aufweist.

4. Zusammensetzung nach einem der Ansprüche 1 bis 3, **dadurch gekennzeichnet, dass** der Komplex der Formel (II) mindestens 94 % des Diastereoisomerenüberschusses, umfassend das Gemisch von Isomeren II-RRR und II-SSS, aufweist.

5. Zusammensetzung nach einem der Ansprüche 1 bis 4, **dadurch gekennzeichnet, dass** sie zwischen 0,01 und 0,3 mol/mol freien makrocyclischen Liganden, bezogen auf den Komplex der Formel (II), umfasst.

6. Zusammensetzung nach einem der Ansprüche 1 bis 5, **dadurch gekennzeichnet, dass** der freie makrocyclische Ligand aus der Gruppe bestehend aus DOTA, NOTA, DO3A, BT-DO3A, HP-DO3A, PCTA, DOTA-GA und Derivaten davon ausgewählt ist.

7. Zusammensetzung nach Anspruch 6, **dadurch gekennzeichnet, dass** es sich bei dem freien makrocyclischen Liganden um 1,4,7,10-Tetraazacyclododecan-1,4,7,10-tetraessigsäure (DOTA) handelt.

8. Zusammensetzung nach einem der Ansprüche 1 bis 7, **dadurch gekennzeichnet, dass** sie eine Konzentration zwischen 0,01 und 1,5 mol.l⁻¹, vorzugsweise zwischen 0,2 und 0,7 mol.l⁻¹, an Komplex der Formel (II) aufweist.

9. Zusammensetzung nach Anspruch 8, **dadurch gekennzeichnet, dass** die Konzentration an Komplex der Formel (II) zwischen 0,3 und 0,6 mol.l⁻¹ liegt.

10. Zusammensetzung nach einem der Ansprüche 1 bis 9, **dadurch gekennzeichnet, dass** ihr pH-Wert zwischen 6,5 und 8 liegt.

11. Zusammensetzung nach einem der Ansprüche 1 bis 10, **dadurch gekennzeichnet, dass** sie den Puffer Tris (Tris(hydroxymethyl)aminomethan) umfasst.

12. Zusammensetzung nach einem der Ansprüche 1 bis 11, **dadurch gekennzeichnet, dass** der Reinheitsgrad des Komplexes der Formel (II) größer als 95 % ist.

13. Zusammensetzung nach einem der Ansprüche 1 bis 12, **dadurch gekennzeichnet, dass** der Reinheitsgrad des Komplexes der Formel (II) größer als 97 % ist.

14. Zusammensetzung nach Anspruch 12 oder 13, **dadurch gekennzeichnet, dass** es sich bei dem freien makrocyclischen Liganden um DOTA handelt und dass sie eine Konzentration an freiem Gadolinium von weniger als 1 ppm (m/v) aufweist.

15. Zusammensetzung nach Anspruch 14, **dadurch gekennzeichnet, dass** sie zwischen 0,002 und 0,4 % mol/mol DOTA, bezogen auf den Komplex der Formel (II), umfasst.

## Claims

1. Composition comprising the complex of formula (II) below: 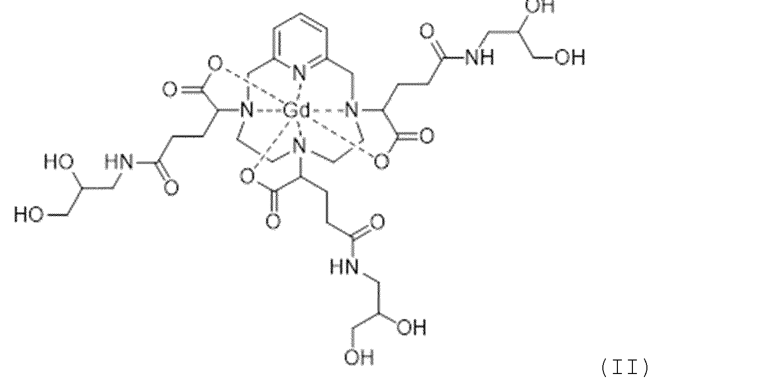 constituted of at least 80% of a diastereoisomeric excess comprising a mixture of isomers II-RRR and II-SSS of formulae: 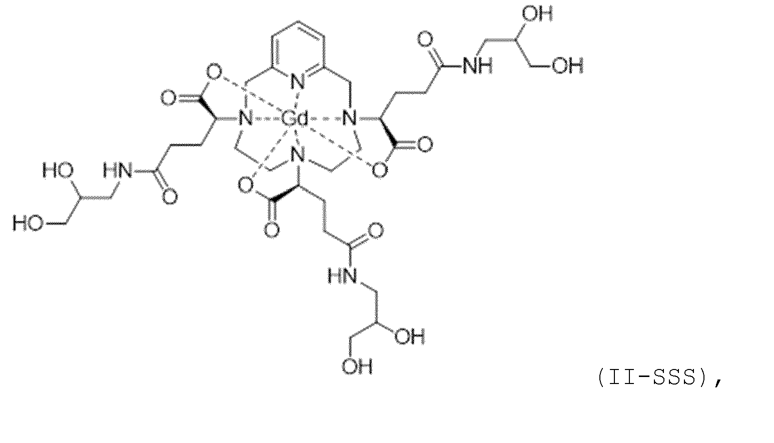 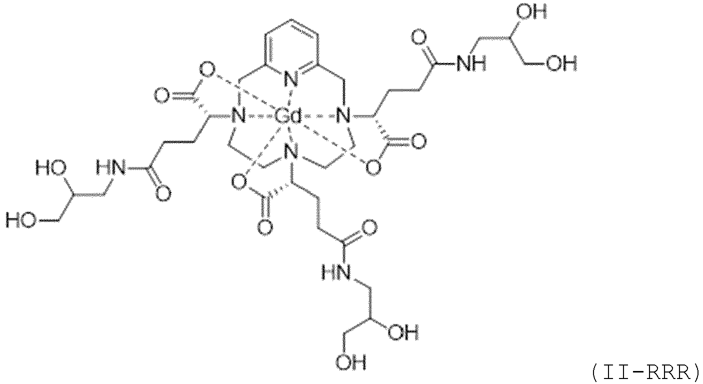 and a free macrocyclic ligand, and advantageously having a concentration of free gadolinium of less than 1 ppm (m/v), **characterized in that** it comprises between 0.002 and 0.4 mol/mol% of free macrocyclic ligand relative to the complex of formula (II).

2. Composition according to Claim 1, **characterized in that** the complex of formula (II) has at least 90% of the diastereoisomeric excess comprising the mixture of isomers II-RRR and II-SSS.

3. Composition according to Claim 1 or 2, **characterized in that** the complex of formula (II) has at least 92% of the diastereoisomeric excess comprising the mixture of isomers II-RRR and II-SSS.

4. Composition according to any one of Claims 1 to 3, **characterized in that** the complex of formula (II) has at least 94% of the diastereoisomeric excess comprising the mixture of isomers II-RRR and II-SSS.

5. Composition according to any one of Claims 1 to 4, **characterized in that** it comprises between 0.01 and 0.3 mol/mol% of free macrocyclic ligand relative to the complex of formula (II).

6. Composition according to any one of Claims 1 to 5, **characterized in that** the free macrocyclic ligand is chosen from the group consisting of DOTA, NOTA, DO3A, BT-DO3A, HP-DO3A, PCTA, DOTA-GA and derivatives thereof.

7. Composition according to Claim 6, **characterized in that** the free macrocyclic ligand is 1,4,7,10-tetraazacyclododecane-1,4,7,10-tetraacetic acid (DOTA).

8. Composition according to any one of Claims 1 to 7, **characterized in that** it has a concentration of between 0.01 and 1.5 mol.L⁻¹, preferably between 0.2 and 0.7 mol.L⁻¹, of complex of formula (II).

9. Composition according to Claim 8, **characterized in that** the concentration of complex of formula (II) is between between 0.3 and 0.6 mol.L⁻¹.

10. Composition according to any one of Claims 1 to 9, **characterized in that** its pH is between 6.5 and 8.

11. Composition according to any one of Claims 1 to 10, **characterized in that** it comprises the buffer Tris (tris(hydroxymethyl)aminomethane).

12. Composition according to any one of Claims 1 to 11, **characterized in that** the degree of purity of the complex of formula (II) is greater than 95%.

13. Composition according to any one of Claims 1 to 12, **characterized in that** the degree of purity of the complex of formula (II) is greater than 97%.

14. Composition according to Claim 12 or 13, **characterized in that** the free macrocyclic ligand is DOTA and **in that** it has a concentration of free gadolinium of less than 1 ppm (m/v).

15. Composition according to Claim 14, **characterized in that** it comprises between 0.002 and 0.4 mol/mol% of DOTA relative to the complex of formula (II).
